(19) 

Europäisches Patentamt

European Patent Office

Office européen des brevets

(11) **EP 3 759 258 B1**

(12) **EUROPEAN PATENT SPECIFICATION**

(45) Date of publication and mention of the grant of the patent:
**13.04.2022 Bulletin 2022/15**

(21) Application number: **18819335.3**

(22) Date of filing: **29.11.2018**

(51) International Patent Classification (IPC):
**C22C 14/00** (2006.01)    **C22F 1/18** (2006.01)

(52) Cooperative Patent Classification (CPC):
**C22C 14/00; C22F 1/183**

(86) International application number:
**PCT/GB2018/053461**

(87) International publication number:
**WO 2019/166749 (06.09.2019 Gazette 2019/36)**

(54) **A BIO-COMPATIBLE TITANIUM ALLOY OPTIMISED FOR ADDITIVE MANUFACTURING**

FÜR DIE GENERATIVE FERTIGUNG OPTIMIERTE BIOKOMPATIBLE TITANLEGIERUNG

ALLIAGE DE TITANE BIOCOMPATIBLE OPTIMISÉ EN VUE DE LA FABRICATION ADDITIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2018 GB 201803176**
**12.09.2018 GB 201814845**

(43) Date of publication of application:
**06.01.2021 Bulletin 2021/01**

(73) Proprietor: **Alloyed Limited**
**Yarnton**
**Kidlington**
**OX5 1QU (GB)**

(72) Inventor: **ALABORT MARTINEZ, Enrique**
**Kidlington, Oxford OX5 1PF (GB)**

(74) Representative: **J A Kemp LLP**
**80 Turnmill Street**
**London EC1M 5QU (GB)**

(56) References cited:
**CN-B- 105 734 312**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to titanium-based alloy compositions designed for enhanced additive manufacturability which has a significantly improved biocompatibility than current alloys and an improved microstructure for strength and toughness. Moreover, the alloy must exhibit a formability window - in terms of cracking susceptibility, solidification range and microstructure stability - which is comparable or wider than equivalent grades of alloy. The mechanical performance at operative conditions have also been weighted in the design process of the new alloy. The Chinese patent application CN 105 734 312 A concerns Ti-alloys that contain additions of Zr, Nb and ta and are intended for use in additive manufacturing processes.

Examples of typical compositions of Ti-alloys which could be used for the manufacture of bio-medical components are listed in Table 1.

Table 1: Nominal composition in wt.% of commercially used titanium alloys for biomedical applications.

| Alloy | Al | V | Fe | Mo | Nb | Ta | Zr | Hf | Sn | Ti |
|---|---|---|---|---|---|---|---|---|---|---|
| Ti-6Al-4V | 6 | 4 | - | - | - | - | - | - | - | Bal. |
| Ti-6Al-7Nb | 6 | - | - | - | 7 | - | - | - | - | Bal. |
| Ti-13Nb-13Zr | - | - | - | - | 13 | - | 13 | - | - | Bal. |
| Ti-12Mo-6Zr-2Fe (TMZF) | - | - | 2 | 12 | - | - | 6 | - | - | Bal. |
| Ti-15Mo-5Zr-3Al | 3 | - | - | 15 | - | - | 5 | - | - | Bal. |
| Ti-12Mo-3Nb | - | - | - | 12 | 3 | - | - | - | - | Bal. |
| Ti-12Mo-5Ta | - | - | - | 12 | - | 5 | - | - | - | Bal. |
| Ti-16N6-10Hf (Tiadyne 1610) | - | - | - | - | 16 | - | - | 10 | - | Bal. |
| Ti-35Nb-7Zr-5Ta (TNZT) | - | - | - | - | 35 | 5 | 7 | - | - | Bal. |
| Ti-29Nb-13Ta-4.6Zr (TNTZ) | - | - | - | - | 29 | 13 | 4.6 | - | - | Bal. |
| Ti-28Nb-13Zr-0.5Fe (TNZF) | - | - | 0.5 | - | 28 | - | 13 | - | - | Bal. |
| Ti-24Nb-4Zr-7.9Sn (Ti2448) | - | - | - | - | 24 | - | 4 | - | 7.9 | Bal. |
| Ti-30Ta | - | - | - | - | - | 30 | - | - | - | Bal. |
| Ti-50Ta | - | - | - | - | - | 50 | - | - | - | Bal. |
| Ti-18Nb-10Ta-2Mo | - | - | - | 2 | 18 | 10 | - | - | - | Bal. |
| Ti-29Nb-13Ta-4Mo | - | - | - | 4 | 29 | 13 | - | - | - | Bal. |
| Ti-16Nb-13Ta-7Mo | - | - | - | 7 | 16 | 13 | - | - | - | Bal. |
| Ti-16Nb-13Ta-4Mo | - | - | - | 4 | 16 | 13 | - | - | - | Bal. |

It is an aim of the invention to provide an alloy suitable for additive manufacturing, preferably which has improved additive manufacturability in comparison with the traditional bio-medical titanium alloys listed in Table 1.

Preferably the alloy will have an improved bio-compatibility (by including mainly elements which are inert, and which promote bone growth) and/or an improved manufacturability (by comparison of the transient solidification behaviour and the phase architecture).

The present invention provides a titanium-based alloy composition consisting in weight percent, of: between 15.0 and 35.0 % niobium, between 0.0 and 7.5 % molybdenum, between 0.0 and 20.0% tantalum, between 0 and 7.0% zirconium, between 0 and 6.0% tin, between 0.0 and 2.0% hafnium, between 0.0 and 0.5 % aluminium, between 0.0 and 0.5 % vanadium, between 0.0 and 0.5 % iron, between 0.0 and 0.5 % chromium, between 0.0 and 0.5% cobalt, between 0.0 and 0.5 % nickel, between 0.0 and 1.0% silicon, between 0.0 and 0.2% boron, between 0.0 and 0.5% calcium, between 0.0 and 0.5% carbon, between 0.0 and 0.5% manganese, between 0.0 and 0.5% gold, between 0.0 and 0.5% silver, between 0.0 and 0.5% oxygen, between 0.0 and 0.5% hydrogen, between 0.0 and 0.5% nitrogen, between 0.0 and 0.5% palladium, between 0.0 and 0.5% lanthanum, the balance being titanium and incidental impurities, wherein the composition satisfies the following equation $0.0175Nb + 0.0183Mo + 0.03Ta + 0.0116Zr + 0.1Sn > 1.0$ where Nb, Mo, Ta, Zr and Sn represent the amounts of niobium, molybdenum, tantalum, zirconium and tin in wt.% respectively. This composition provides a good balance between biocompatibility, cracking susceptibility during manufacturing, optimal mechanical performance at service temperature and stable microstructure during solidification.

In an embodiment of the invention, the titanium alloy consists of 7.0% or less molybdenum, preferably 6.0% or less molybdenum, more preferably 5.0 or less molybdenum. This alloy has improved properties for additive manufacturability due to a lower solidification range and reduced martensitic start temperature as molybdenum deleteriously effects these properties.

In an embodiment of the invention, the titanium alloy consists of 1.5% or more, preferably 2.5% or more molybdenum. Molybdenum is beneficial because of its higher growth restriction factor (and resulting finer microstructure). This amount of molybdenum also reduces the martensitic start temperature of the composition while using small wt. % of alloyants. Even a low level of molybdenum of 1.5% or more has a significant effect in this regard and use of molybdenum to achieve a low martensitic start temperature is preferred to other candidate alloyants which are less effective beta stabalisers.

In an embodiment, the titanium alloy satisfies the following equation $0.0375Nb + 0.033Mo + 0.0167Ta + 0.05Zr + 0.267 - (0.13Sn - 0.516)^2 > 1.0$ where Zr, Sn, Mo, Ta and Nb represent the amounts of zirconium, tin, molybdenum, tantalum and niobium in wt. % respectively. Such an alloy has improved crack susceptibility factor meaning additive manufacturability is improved.

In an embodiment the alloy satisfies $0.0178Nb + 0.0143Mo + 0.0243Ta + 0.0285Sn < 1.0$. Such an alloy is easier to powder process due to its low melting temperature.

In an embodiment the alloy satisfies $0.0298Nb + 0.0272Mo + 0.0246Ta + 0.0376Zr + 0.0259Sn > 1.0$ such an alloy has improved biocompatibility.

In an embodiment the alloy satisfies $75 > 883 - 150Fe - 49Mo - 17Nb - 12Ta - 7Zr - 3Sn < 250$. Such an alloy has a martensitic start temperature which does not compromise the manufacturing process but can be used to add strength, shape memory effect and to lower the stiffness of the alloy.

In an embodiment the alloy satisfies $2.5 > 0.042Nb + 0.06Mo + 0.05Ta + 0.03Zr + 0.1Sn > 1.0$. Such an alloy has an optimal freezing range which is desirable during laser manufacturing.

In an embodiment of the invention, the titanium alloy consists of 17.5% or more niobium, preferably 20.0% or more niobium, more preferably 22.5% or more niobium. More niobium achieves a lower cracking susceptibility factor and so excellent additive manufacturing properties. Niobium also increases the bonding order so the elastic modulus is reduced.

In an embodiment of the invention, the titanium alloy consists of 32.5% or less niobium, preferably 30.0% or less niobium, more preferably 27.5% or less niobium. Reducing the amount of niobium reduces the cost of the alloy whilst increasing strength and ease of manufacture of powder.

In an embodiment of the invention, the titanium alloy consists of 17.5% or less tantalum, more preferably 15.0% or less tantalum. Higher amounts of Ta can make the production of ingots difficult, particularly when melting directly from elementals. This also reduces the cost of the alloy and also makes the process for producing the alloy melt easier because tantalum has an extremely high melting temperature.

In an embodiment of the invention, the titanium alloy consists of 5.0% or more tantalum, preferably 7.5% or more tantalum, more preferably 10.0% or more tantalum, most preferably 12.5% or more tantalum. Such an alloy has a high bonding order, indicating good biocompatibility and low stiffness.

In an embodiment of the invention, the titanium alloy consists of 6.0% or less zirconium, preferably 5.5% or less, more preferably 5.0% or less zirconium even more preferably 4.5% or less zirconium. Less zirconium reduces the cost of the alloy and increases the amount of titanium for powder processability.

In an embodiment of the invention, the titanium alloy consists of 1.0% or more zirconium preferably 1.5% or more zirconium, more preferably 2.0% or more zirconium. Increasing zirconium content improves the strength (by solid solutioning and grain refinement) and reduces the stiffness without greatly increasing the solidification range for optimal manufacturing. Zirconium also advantageously improves bio-compatability and also inhibit the formation of omega phase which is otherwise deleterious to mechanical properties and superelastic/shape-memory behaviour E. L. Pang et al. "The effect of zirconium on the omega phase in Ti-24Nb [0-8] Zr alloys", Acta Materialia, Vol. 153, pp 62-70.

A combination of a minimum level of 1.5% molybdenum and a minimum level of 1.0% zirconium is particularly preferable because such alloy will form the martensite phase at low temperature - while requiring low amounts of beta stabilisers - and during the process it will avoid the formation of omega phase due to the Zr addition. Higher levels of zirconium and/or molybdenum than the 1.0% and 1.5% respectively are even more preferred to further metastabilise the alloy and to improve both the microstructural stability of the alloy but also to increase the bio-compatibility.

In an embodiment of the invention, the titanium alloy satisfies the flowing equation

$$250 \geq 883 - 150\,Fe\,wt.\% - 96\,Cr\,wt.\% - 49\,Mo\,wt.\% - 37\,V\,wt.\% - 17\,Nb\,wt.\% - 12\,Ta\,wt.\% - 7\,Zr\,wt.\% + 15\,Al\,wt.\%,$$

preferably

$$225 \geq 883 - 150\, Fe\, wt.\% - 96\, Cr\, wt.\% - 49\, Mo\, wt.\% - 37\, V\, wt.\% -$$

$$17\, Nb\, wt.\% - 12\, Ta\, wt.\% - 7\, Zr\, wt.\% - 3\, \text{Sn wt.}\% + 15\, Al\, wt.\%,$$

more preferably

$$200 \geq 883 - 150\, Fe\, wt.\% - 96\, Cr\, wt.\% - 49\, Mo\, wt.\% - 37\, V\, wt.\% - 17\, Nb\, wt.\%$$

$$- 12\, Ta\, wt.\% - 7\, Zr\, wt.\% - 3\, \text{Sn wt.}\% + 15\, Al\, wt.\%$$

in which Fe, Cr, Mo, V, Nb, Ta, Zr, Sn and Al represent the amounts of iron, chromium, molybdenum, vanadium, niobium, tantalum, zirconium, tin and aluminium in wt% respectively. Such an alloy is unlikely to compromise the manufacturing process due to the formation of brittle martensite phase during the thermal cycling which occurs during manufacturing. The lower the martensite start temperature the better and martensite start temperatures of 225°C or less and even more preferably 200°C or less is desirable.

In an embodiment of the invention, the titanium alloy satisfies the flowing equation

$$75 \leq 883 - 150\, Fe\, wt.\% - 96\, Cr\, wt.\% - 49\, Mo\, wt.\% - 37\, V\, wt.\% - 17\, Nb\, wt.\%$$

$$- 12\, Ta\, wt.\% - 7\, Zr\, wt.\% - 3\, \text{Sn wt.}\% + 15\, Al\, wt.\%. \leq 250$$

in which Fe, Cr, Mo, V, Nb, Ta, Zr, Sn and Al represent the amounts of iron, chromium, molybdenum, vanadium, niobium, tantalum, zirconium, tin and aluminium in wt% respectively. Such an alloy is unlikely to compromise the manufacturing process because the martensite transformation occurs at low temperatures. Moreover, because such an alloy has martensitic phase at room temperature, it offers improved strength, shape memory effect, and thus the possibility of even lower apparent stiffness.

In an embodiment of the invention, the titanium alloy consists of 50.0 wt% titanium or more, preferably 52.5 wt% or more titanium, more preferably 55.0 wt% or more titanium. A powder of such an alloy is easier to produce with good chemical homogeneity and reduced segregation.

In an embodiment of the invention, the titanium alloy consists of 6.5% or less tin, preferably 6.0% or less tin, preferably 5.5% or less tin. This ensures that the alloy is minimally $\alpha$-stabilised, that the martensitic start temperature stays within range and that the cracking susceptibility stays low.

In an embodiment of the invention, the titanium alloy consists of 2.0% or more tin, preferably 3.0% or more tin, more preferably 4.0% or more tin. This improves the strength of the alloy whilst reducing stiffness and ensures that the cracking susceptibility stays close to the optimal.

In an embodiment of the invention, the titanium alloy satisfies the flowing equation

$$1.0 \leq Al\, \text{wt.}\% + \frac{1}{3}\, \text{Sn wt.}\% + \frac{1}{6}\, \text{Zr wt.}\% \leq 2.5$$

in which, Al, Sn and Zr represent the amounts of aluminium, tin and zirconium in wt% respectively. Such an alloy has increased strength of the alpha phase which may appear at room temperature. Since Al is restricted, there is a need to add Sn and Zr in Al equivalent wt. % values between 1.0 and 2.5 wt. %.

In an embodiment of the invention, the titanium alloy consists of 0.1 wt% or more hafnium, preferably 0.5 wt% or more hafnium as this increases wear resistance by increasing the hardness.

In an embodiment of the invention the sum of wt % of each of aluminium, vanadium, iron, chromium, cobalt, nickel and manganese is 1.0 wt.% or less, preferably 0.5wt% or less. Such an alloy has a low level of elements with low biocompatibility.

The term "consisting of" is used herein to indicate that 100% of the composition is being referred to and the presence of additional components is excluded so that percentages add up to 100%. Unless otherwise stated, all amounts are in weight percent.

The invention will be more fully described, by way of example only, with reference to the accompanying drawings in which:

Figure 1 illustrates the biocompatibility of various alloying elements;

4

Figure 2 is a flow diagram illustrating the process by which the titanium-based alloy composition was determined;

Figure 3 illustrates the contour plots for the crack susceptibility factor (CSF) for the Ti-Nb-Mo-Ta-Zr system;

Figure 4 illustrates the contour plots for the Scheil solidification (freezing) range for the Ti-Nb-Mo-Ta-Zr system;

Figure 5 illustrates the contour plots for the cost of the Ti-Nb-Mo-Ta-Zr system;

Figure 6 illustrates the contour plots for the martensitic start temperature of the Ti-Nb-Mo-Ta-Zr system;

Figure 7 illustrates the contour plots for the bonding order of the Ti-Nb-Mo-Ta-Zr system;

Figure 8 illustrates the contour plots for the growth restriction factor (GRF) of the Ti-Nb-Mo-Ta-Zr system;

Figure 9 illustrates the contour plots for the melting temperature of the Ti-Nb-Mo-Ta-Zr system;

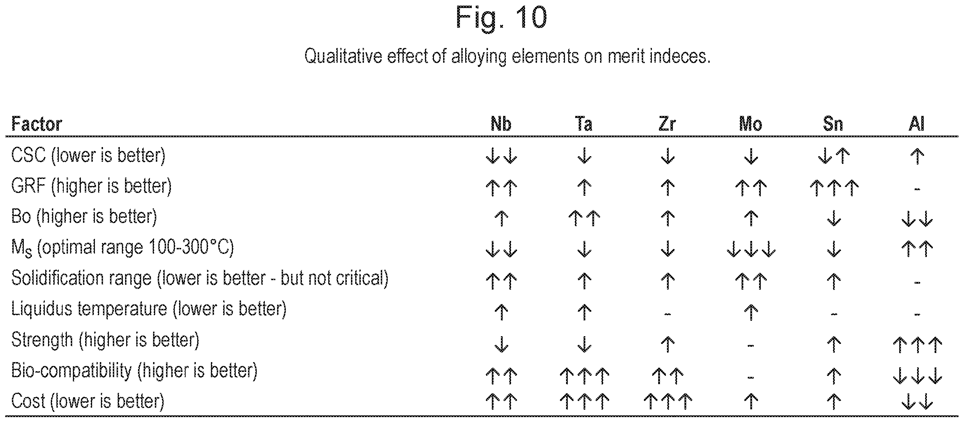

Figure 10 illustrates qualitatively the effect of each element on the proposed merit indices;

Figure 11 illustrates the constrains used to isolate an alloy space with merit indices comparable to or better than the current alloys;

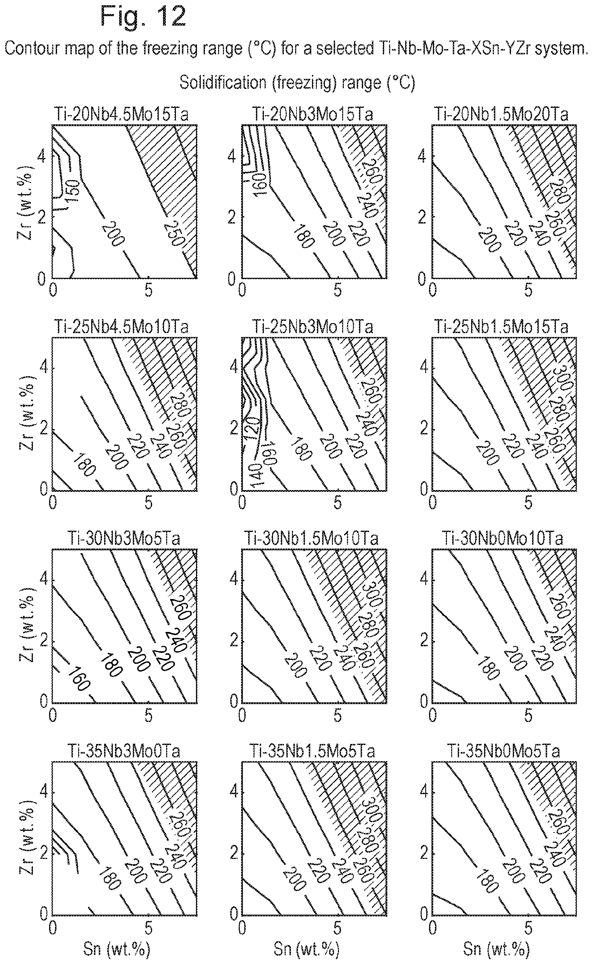

Figure 12 illustrates the effect of Sn and Zr on the contour plots for the solidification range of a few selected Ti-Nb-Mo-Ta systems;

Figure 13 illustrates the effect of Sn and Zr on the contour plots for the cracking susceptibility factor of a few selected Ti-Nb-Mo-Ta systems;

Figure 14 illustrates the effect of Sn and Zr on the contour plots for the growth restriction factor of a few selected Ti-Nb-Mo-Ta systems;

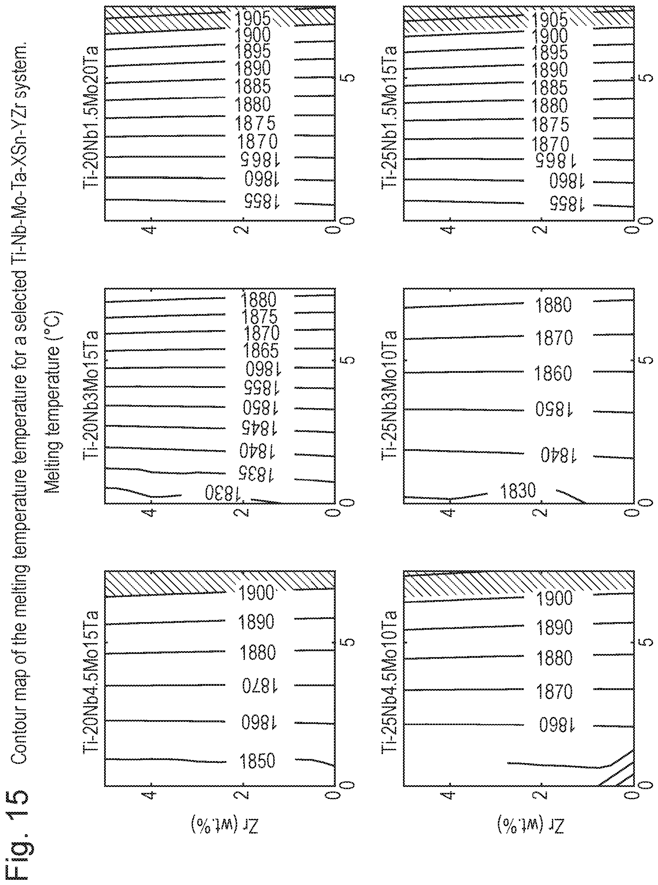

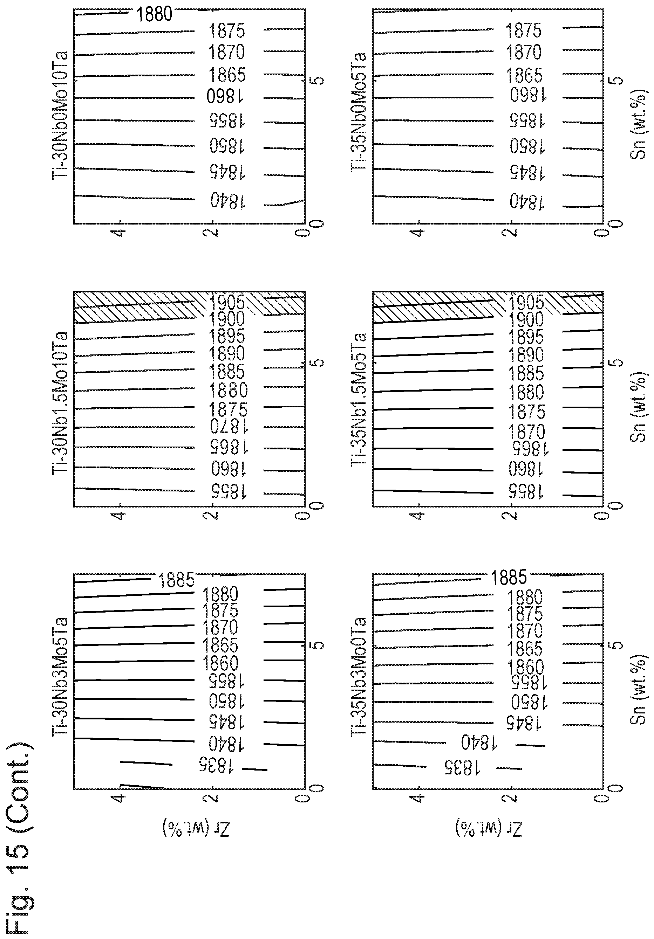

Figure 15 illustrates the effect of Sn and Zr on the contour plots for the melting temperature of a few selected Ti-Nb-Mo-Ta systems;

Figure 16 illustrates the effect of Sn and Zr on the contour plots for the martensite start temperature of a few selected Ti-Nb-Mo-Ta systems;

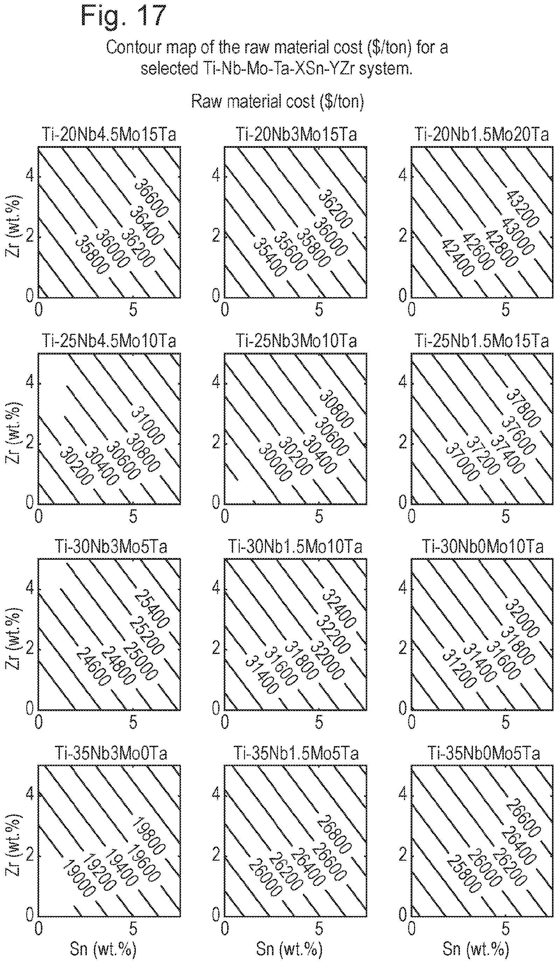

Figure 17 illustrates the effect of Sn and Zr on the contour plots for the raw material cost of a few selected Ti-Nb-Mo-Ta systems;

Figure 18 illustrates the effect of Sn and Zr on the contour plots for the bond order of a few selected Ti-Nb-Mo-Ta systems;

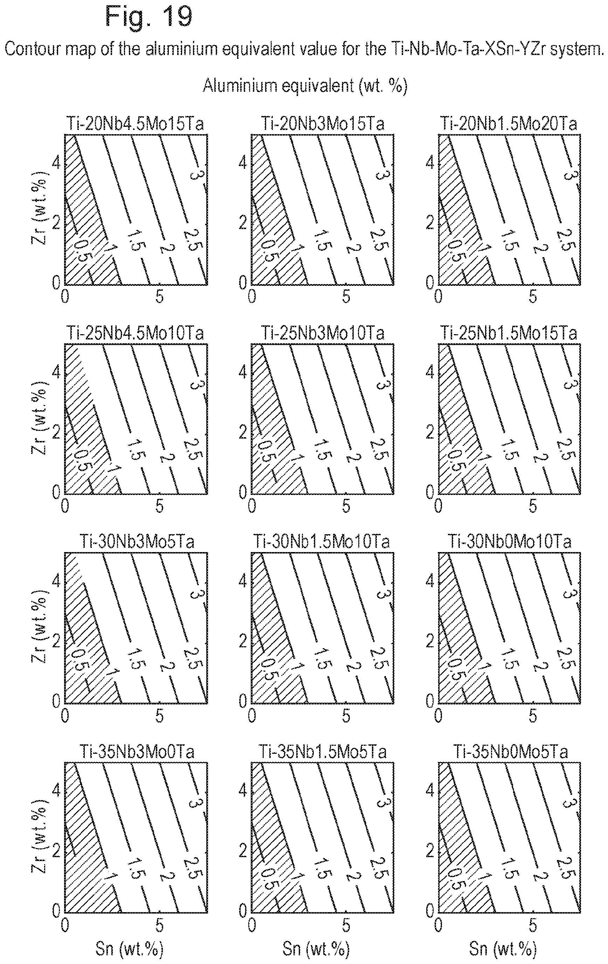

Figure 19 illustrates the effect of Sn and Zr on the contour plots for the aluminium equivalent of a few selected Ti-Nb-Mo-Ta systems;

Figure 20 illustrates the optimal alloy space as constrained by the threshold equations;

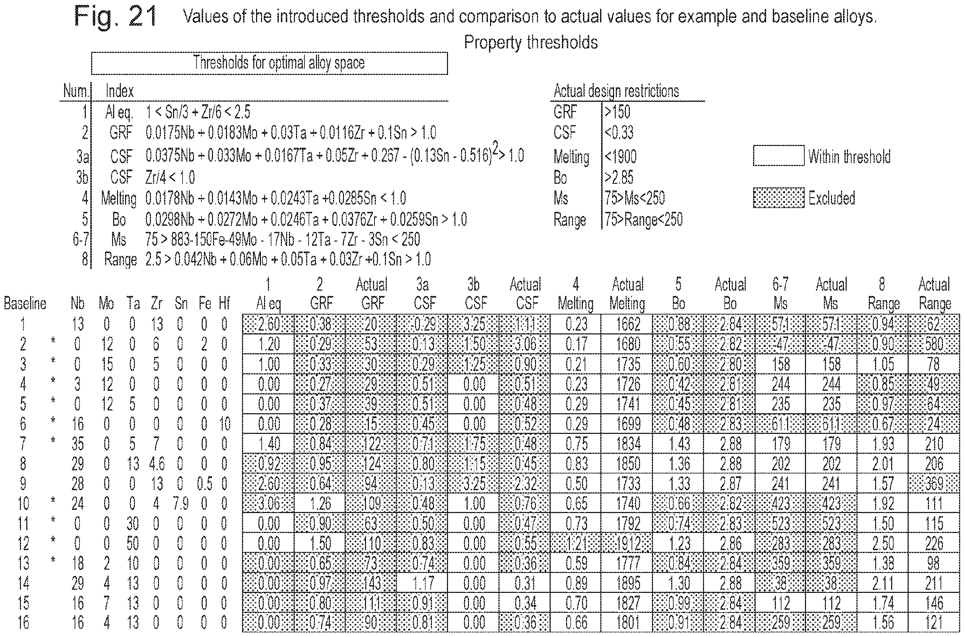

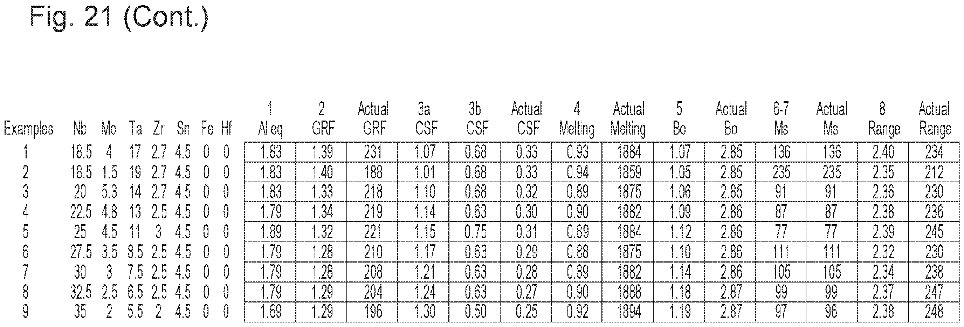

Figure 21 illustrates the values of the threshold equations for each of the examples and baseline alloys;

Figure 22 illustrates the scatter plots of the solidification range vs. growth restriction factor. Area highlighted in black indicates the optimal design space. Labelled data points correspond to literature alloys. Grey indicates the rest of the trial compositions;

Figure 23 illustrates the scatter plots of the solidification range vs. cracking susceptibility factor. Area highlighted in black indicates the optimal design space. Labelled data points correspond to literature alloys. Grey indicates the rest of the trial compositions;

Figure 24 illustrates the scatter plots of the cracking susceptibility vs. growth restriction factor. Area highlighted in black indicates the optimal design space. Labelled data points correspond to literature alloys. Grey indicates the rest of the trial compositions;

Figure 25 illustrates the scatter plots of the growth restriction factor vs. other merit indices. Area highlighted in black indicates the optimal design space. Labelled data points correspond to literature alloys. Grey indicates the rest of the trial compositions;

Figure 26 illustrates the scatter plots of the cracking susceptibility factor vs. other merit indices. Area highlighted in black indicates the optimal design space. Labelled data points correspond to literature alloys. Grey indicates the rest of the trial compositions;

Traditionally, titanium-based alloys have been designed through empiricism. Thus, their chemical compositions have been isolated using time consuming and expensive experimental development, involving small-scale processing of limited quantities of material and subsequent characterisation of their behaviour. The alloy composition adopted is then the one found to display the best, or most desirable, combination of properties. The large number of possible alloying elements indicates that these alloys are not entirely optimised and that improved alloys are likely to exist.

In titanium alloys, generally additions of aluminium (Al) are added as $\alpha$-stabiliser to improve the mechanical strength. However, aluminium has been associated with neurological related diseases. General additions of vanadium (V) are added as $\beta$-stabiliser and to increase the mechanical strength without forming brittle intermetallic compounds. V makes a solid solution with the $\beta$ phase. However, V is believed to have toxic and carcinogenic effects on the body. Additions of nickel (Ni), cobalt (Co), iron (Fe) and chromium (Cr) are also added as $\beta$-stabiliser elements. However, all of these are considered to have low bio-compatibility and are associated with adverse responses to body functions. Therefore, the alloy contains a maximum amount of 0.5% of each of Al, V, Ni, Co, Mn, Fe and Cr preferably 0.2% or less of each of Al, V, Ni, Co, Mn, Fe and Cr and a total sum of these elements and boron of 1.0% or less, preferably 0.5% or less.

Calcium and carbon may be present at levels of up to 0.5% each and are not expected greatly to change the character of the alloy at this level.

Oxygen, nitrogen and hydrogen may be present in the alloy at concentrations up to 0.5wt% each without reducing the characteristics of the alloy significantly. Oxygen will significantly improve the strength of the alloy but high amounts may have a negative effect on any shape memory effects that the alloy may exhibit. The same is true of gold, silver and palladium, each of which may be present in an amount of up to 0.5wt%. These may be added to increase bio-compatibility and to reduce infections. Lanthanum may be added in an amount up to 0.5wt.% in order to further improve the microstructure during solidification (similarly to B).

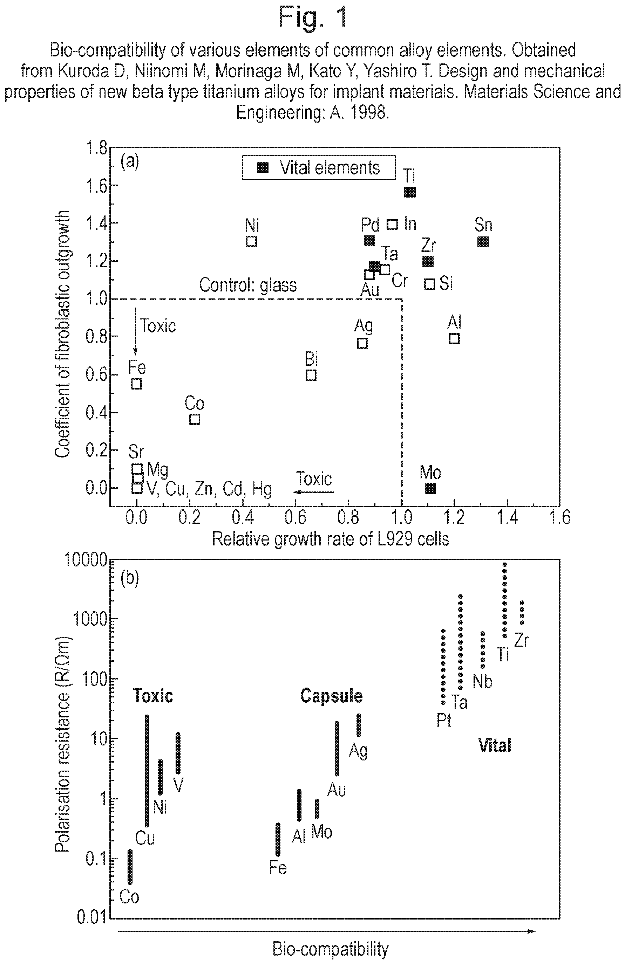

Pure titanium, which is biocompatible has poor solidification characteristics (in particular crack susceptibility factor, CSF), discussed below. Therefore, alloying elements are added in the present invention to improve the additive manufacturability of the alloy. Because the alloy is intended for use in an article to be placed in the human or animal body including prosthetic devices, orthopaedic implants, in particular bone implants and/or artificial joints, it is desirable to achieve the desired properties of the titanium alloy using mainly or even exclusively so-called "vital" elements which have been shown to have high biocompatibility and osseo-integration. Figure 1 is a graph showing the biocompatibility or otherwise of various possible alloying elements, obtained from Kuroda D, Niinomi M, Morinaga M, Kato Y, Yashiro T. Design and mechanical properties of new $\beta$ type titanium alloys for implant materials. Materials Science and Engineering: A. 1998.

In the alloy of the present invention, niobium (Nb) is used as $\beta$-stabiliser and to lower the elastic modulus of the alloy. Tantalum (Ta) is also used as $\beta$-stabiliser and to promote bone growth into the metal. Zirconium (Zr) is used as replacement of Ti further to reduce the elastic modulus - in combination with Nb and Ta, Zr can perform as $\beta$-stabiliser.

In the present invention, molybdenum (Mo) is used as a strong $\beta$-stabiliser to improve the properties during manufacturing. Although Mo is not a so-called 'vital' element, it has been shown that, when combined with Ti, it does not degrade the bio-compatibility of the alloy and that it has no adverse effects on the body - see Nunome et. al. In vitro evaluation of biocompatibility of Ti-Mo-Sn-Zr superelastic alloy, Journal of Biomaterials Applications, 2015.

Tin (Sn) is used to reduce the stiffness of the alloy and to add strength. Because Sn is an $\alpha$-stabiliser, the amount of Sn is limited to Sn $\leq$ 7.0 wt. %, preferably 6.0% or less, more preferably 5.5% or less. In an embodiment at least 2.5% or more Sn is added to reduce stiffness, to increase strength and to obtain the most optimal crack susceptibility. More preferably there is 3.0% or more tin, yet further to reduce stiffness and increase strength. It is assumed that Sn can replace Zr in the present invention. A minimum of 4.0 % tin is desirable for reduce stiffness and increased strength.

In an embodiment of the invention, the titanium alloy satisfies the flowing equation

$$1.0 \leq \text{Al wt.} \% + \frac{1}{3}\text{Sn wt.} \% + \frac{1}{6}\text{Zr wt.} \% \leq 2.5$$

Tin and Zr may be interchangeable and when present in a preferable amount of 1.0% Al. eq. or more as illustrated by the above equation to increase the strength of the alloy and reduce the overall stiffness while improving on bio-compatibility. A preferable maximum amount of 2.5% Al. eq. ensures that the solidification range stays within acceptable limits while still obtaining the bio-compatibility benefits that Ta, Nb and Mo add while keeping a minimum amount of 52.5% of pure titanium in the final composition.

Hafnium (Hf) can optionally be used to increase the hardness of the alloy. However, Hf is a very expensive element and its used is restricted in the present invention to Hf $\leq$ 2.0 wt. %. Preferably Hf is present at a level of 1.0% or less to keep cost down. For applications where high hardness (to improve wear resistance) is important, Hf can be present in an amount of 0.1% or more preferably 0.5% or more.

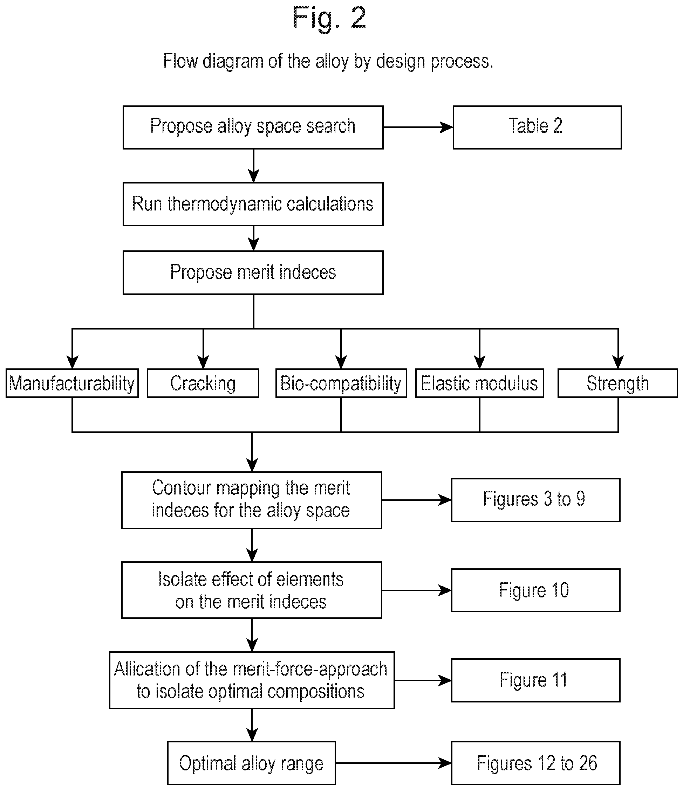

A modelling-based approach used for the isolation of new grades of titanium-based bio-compatible alloys is described here, termed the "Alloys-By-Design" (ABD) method. This approach utilises a framework of computational materials models to estimate design relevant properties across a very broad compositional space. Figure 2 illustrates the various steps of the method. In principle, this alloy design tool allows the so-called inverse problem to be solved; identifying optimum alloy compositions that best satisfy a specified set of design constraints.

The first step in the design process is the definition of an elemental list along with the associated upper and lower compositional limits. The compositional limits for each of the elemental additions considered in this invention - referred to as the "alloy design space" - are detailed in Table 2.

Table 2: Alloys design space in wt.% searched using the 'Alloy-by-Design' method.

|  | Nb | Mo | Ta | Zr | Sn |
|---|---|---|---|---|---|
| **Min.** | 0.0 | 0.0 | 0.0 | 0.0 | 0.0 |
| **Max.** | 40.0 | 15.0 | 30.0 | 20.0 | 12.5 |

The second step relies upon thermodynamic calculations used to calculate the phase diagram and thermodynamic properties for a specific alloy composition. Often this is referred to as the CALPHAD method (CALculate PHAse Diagram). These calculations are conducted for those temperatures where an optimal phase architecture of the new alloy is found.

A third stage involves isolating alloy compositions which have the desired microstructural architecture for additive manufacturing and for bio-medical purposes. In the case of titanium alloys, the formability *via* additive manufacturing is related directly to the weldability of the alloy. In titanium alloys, the weldability can be correlated in a first instance to the microstructural architecture in terms of $\alpha$ and $\beta$ phase proportions. For near-$\alpha$ alloys, the weldability is good, these alloys are usually welded in annealed condition. For the $\alpha/\beta$ alloys, weldability is dependent on the amount of $\beta$ phase present. The most strongly beta stabilised alloys are usually embrittled during welding - high beta content alloys are rarely welded. The exception is Ti-6Al-4V which has good weldability and can have good mechanical properties after heat-treatment. Metastable $\beta$ alloys have good weldability and retain good mechanical properties after welding even without the need of post-heat treatment. In the bio-medical field, there is a need to design alloys that have low elastic modulus (to be close to that of the bone) and to use alloyants that improve bio-compatibility and osseo-integration. Metastable $\beta$ alloys (BCC) have been shown to decrease substantially the elastic modulus when compared to $\alpha$ and $\beta$ alloys (HCP + BCC). Moreover, the use of Nb, Zr, and Ta (all of them $\beta$ stabilisers) is known to improve the bio-compatibility and osseo-integration of titanium alloys. Thus, it is desirable for the purposes here exposed to design a metastable $\beta$ alloy employing the so-called 'vital' elements.

Thus, the model isolates all compositions in the design space which are the most bio-compatible, which tend to form stable $\beta$ microstructures for optimal additive manufacturability, and which have a low elastic modulus for good osseo-integration and reduced stress-shielding. Moreover, the important factors such as melting temperature and amount of alloyants - which are important to obtain a uniform and chemically homogeneous powder particle - are also weighted into the design process.

In the fourth stage, merit indices are estimated for the remaining isolated alloy compositions in the dataset. Examples of these include: martensitic transformation merit index (which describes the temperature at which the transformation starts), elastic modulus and bone-growth compatibility (which are related to the bond order of the composition), manufacturability (which is related to the freezing range and the susceptibility of the alloy to crack which is a function of the transient solidification behaviour and the phase proportions of the alloy), and powder processability (which is related to the melting temperature and the amount of titanium in the alloy).

The first merit index is the bone-compatibility merit index, which highlights the speed at which cells grow into the metal interface. Okazaki and Tetsuya. (1998), Corrosion resistance, mechanical properties: corrosion fatigue strength and cytocompatibility of new Ti alloys without Al and V. Biomaterials, has shown that the bone-growth correlates with the bonding order of the alloy. Thus, the merit index is calculated using a rule of mixtures following

$$Bo = \sum_i x_i Bo_i$$

where $x_i$ is the concentration of element $i$ and $Bo_i$ the $ith$ bonding order value of the element. Moreover, this merit index also measures the critical current density for passivation (which gives a measurement of the cytotoxicity) and an approximation to the elastic modulus of the alloy. Increasing the bonding order helps decreasing both the cytotoxicity and the elastic stiffness - see Brown SA, Lemons JE (1996) Medical Applications of Titanium and Its Alloys: The Material and Biological Issues.

*Table 3: Bonding order of each element.*

| Ti | V | Fe | Ni | Zr | Nb | Mo | Hf | Ta | Al | Si | Sn |
|---|---|---|---|---|---|---|---|---|---|---|---|
| 2.79 | 2.8 | 2.65 | 2.41 | 3.08 | 3.09 | 3.06 | 3.11 | 3.14 | 2.42 | 2.56 | 2.28 |

The second merit index relates to the susceptibility of the alloy to form the martensitic phase. This merit index uses the martensite start temperature model as disclosed in Suresh Neelakantana, Prediction of the martensite start temperature for β titanium alloys as a function of composition, 60 Scripta Materialia 611 (2009). The martensitic start temperature (°C) is calculated as a function of the alloy composition following

$$M_S = 883 - 150\,Fe\,wt.\% - 96\,Cr\,wt.\% - 49\,Mo\,wt.\% - 37\,V\,wt.\% - 17\,Nb\,wt.\%$$
$$- 12\,Ta\,wt.\% - 7\,Zr\,wt.\% - 3\,Sn\,wt.\% + 15\,Al\,wt.\%.$$

If the martensitic start temperature is below ambient temperature, martensitic phase will not be present at the microstructure level. Martensitic phase is hard and brittle, and it could affect the crack susceptibility of the alloy if the thermal strains during the manufacturing process are high enough to crack this brittle phase. A preferable upper limit of the martensitic start temperature is defined so that the phase transformation does not compromise the manufacturing process ($\sim \leq 250$°C). A reduction in the likelihood of martnesite formation is achieved by reducing the martensite start temperature even further. Therefore, a martensite start temperature of 225°C or less is preferred and the most desirable alloys have a martensite start temperature of 200°C or less. A lower limit is also defined, a martensitic transformation above the service temperature can be used to add strength, shape memory effect, and to further lower the stiffness of the alloy ($\sim \geq 75$°C). The optimal martensitic start temperature is regarded as being between 75 to 200°C.

The third merit index is the freezing range. The freezing range is effectively the temperature range of the two-phase liquid + β phase region.

This merit index is calculated using the Scheil thermodynamic calculation in ThermoCalc and provides the transient solidification route of the alloy. An expanded solidification range has the potential to increase hot tearing and remelting of underneath layers during laser manufacturing. Thus, it is desirable to monitor the temperature range at which the transition from liquid to solid occurs. Although not the most critical factor, it is desirable to minimise the solidification range, at least to a value comparable to those alloys in the literature.

The fourth merit index is the crack susceptibility factor (CSF), it is calculated as a function of the time needed to solidify the last 10% of the liquid versus the time necessary to go from a 40% solid fraction to 90% solid fraction. The solidification is assumed to occur at a constant rate of decrease in temperature in order to facilitate the estimation of the solidification time. It is assumed that the risk of hot tearing is stronger during the last instants of the solidification, thus the goal is to minimise the time of that part of the solidification process. The CSF is calculated following

$$CSF = \frac{\text{time}(f_{solid} = 0.9) - \text{time}(f_{solid} = 1.0)}{\text{time}(f_{solid} = 0.4) - time(f_{solid} = 0.9)}$$

where time($f_{solid}$) is the time required to reach a particular volume fraction of solid.

The fifth merit index is the growth restriction factor (GRF). The methodology to derive it is described in T. E. Quested, A. T. Dinsdale Et A. L. Greer, Thermodynamic Modeling of Growth-Restriction Effects in Aluminum Alloys, 53 Acta Materialia 1323 (2005). Generally, it is accepted that an alloy with a large growth restriction factor tends to reject solute atoms during solidification which translate into finer grain size microstructures which are beneficial for additive manufacturing. The GRF is calculated as the derivative of the fraction of solid with respect to undercooling following

$$\mathrm{GRF} = \left( \frac{\partial(\Delta T_S)}{\partial f_s} \right)_{f_s \to 0}$$

where $f_s$ is the fraction of solid and $\Delta T_S$ is the solutal undercooling. This can be approximated using the Scheil analysis by calculating the slope of a linear regression fit to the temperature profile as a function of the solid fraction for solid fractions below 0.1. Further details can be found in the aforementioned scientific reference.

The sixth merit index is the powder processability. In order to facilitate the powder production process and obtain optimal elemental homogenisation, the melting temperature and the amount of alloyants must be minimised. Thus, for the sixth index, the temperature of the melting point is calculated, this is desirably below 1900°C. The sixth merit index also limits the amount of alloyants. Preferably pure titanium is at least 50.0 wt. % of the final composition. It is thought that it is desirable to maintain titanium in at least an amount of 52.5 wt. %, preferably at least 55.0 wt.% of the final composition to ease powder processing. If too little titanium is present it is believed that it may make processing of the powder (produced by atomisation of an ingot) difficult. That is, when the ingot of the correct composition is atomised, if too little titanium is present this may cause segregation whose occurrence is also related to the melting point. The atomised powder is then fed into a selective laser melting (SLM) machine for additive manufacture.

The ABD method described above was used to isolate the inventive alloy composition. The design intent for this alloy was to isolate the composition of a new titanium alloy which exhibits a combination of stiffness, strength, manufacturability, processability, cytotoxity and osseo-integration which is comparable or better than equivalent grades of alloy. The cost of the alloy has also been considered in the design of the new alloy. The cost is based on the amount of each element contained in the alloy at 2018 prices.

The material properties - determined using the ABD method - for the literature titanium alloys are listed in Table 4. The design of the new alloy was considered in relation to the predicted properties listed for these alloys. The method was used to propose nine optimised alloy compositions which target different properties: ranging from highest GRF to lowest GRF and lowest CSC to highest CSC respectively. The calculated material properties for the optimised alloys with nominal compositions according to Table 5 and in accordance with the present invention are also given.

**Table 4:** Calculated phase fractions and merit indices made with the "Alloys-by-Design" software. Results for four commonly used biomedical Ti alloys as listed in Table 1 and the nominal composition of the new alloys listed in Table 5.

| | ID | Freezing (°C) | CSF (-) | GRF (-) | Bo (-) | Ms (°C) | Vital (-) | Weldability (-) | Melting (-) | Al eq. (wt. %) | Ti (-) | Cost (S/ton) |
|---|---|---|---|---|---|---|---|---|---|---|---|---|
| Ti-6Al-4V | 1 | 27 | 1.31 | 5.5 | 2.75 | 825 | No | $\alpha+\beta$ | 1703 | 6.0 | 90 | 5529 |
| Ti-6Al-7Nb | 2 | 26 | 1.06 | 7.3 | 2,76 | 854 | No | $\alpha+\beta$ | 1717 | 6.0 | 87 | 7170 |
| Ti-13Nb-13Zr | 3 | 62 | 1.11 | 20.0 | 2.84 | 571 | Yes | $\alpha+\beta$ | 1662 | 2.2 | 74 | 28447 |
| Ti-12Mo-6Zr-2Fe (TMZF) | 4 | 580 | 3.06 | 53.2 | 2,82 | -47 | No | Metastable $\beta$ | 1680 | 1.0 | 80 | 14136 |
| Ti-15Mo-5Zr-3Al | 5 | 78 | 0.90 | 30.4 | 2.80 | 158 | No | Metastable $\beta$ | 1735 | 3.8 | 77 | 12860 |
| Ti-12Mo-3Nb | 6 | 49 | 0.51 | 28.8 | 2.81 | 244 | Yes | Metastable $\beta$ | 1726 | 0.0 | 85 | 6621 |
| Ti-12Mo-5Ta | 7 | 64 | 0.48 | 39.0 | 2.81 | 235 | Yes | Metastable $\beta$ | 1741 | 0.0 | 83 | 12830 |
| Ti-16Nb-10Ht (Tiadyne 1610) | s | 24 | 0.52 | 14.8 | 2.83 | 541 | Yes | $\beta$ (martensitic) | 1699 | 0.0 | 74 | 110192 |
| Ti-35Nb-7Zr-5Ta (TNZT) | 9 | 210 | 0,48 | 122.5 | 2.88 | 179 | Yes | Metastable $\beta$ | 1834 | 1.2 | 53 | 35119 |
| Ti-29Nb-13Ta-4.6Zr (TNTZ) | 10 | 206 | 0.45 | 124.4 | 2.88 | 202 | Yes | Metastable $\beta$ | 1850 | 0.8 | 53 | 41128 |
| Ti-28Nb-13Zr-0.5Fe (TNZF) | 11 | 369 | 2.32 | 93.7 | 2.87 | 241 | No | Metastable $\beta$ | 1733 | 2.2 | 59 | 33928 |
| Ti-24Nb-4Zr-7.9Sn (Ti2448) | 12 | 111 | 0.76 | 109.9 | 2.82 | 423 | Yes | $\beta$ (martensitic) | 1740 | 3.3 | 64 | 20696 |
| Ti-30Ta | 13 | 115 | 0.47 | 63.3 | 2.83 | 523 | Yes | $\beta$ (martensitic) | 1792 | 0.0 | 70 | 48660 |
| Ti-50Ta | 14 | 226 | 0.55 | 109.9 | 2.86 | 283 | Yes | $\beta$ (martensitic) | 1912 | 0.0 | 50 | 77900 |
| *Ti-18Nb-10Ta-2Mo* | 15 | 98 | 0.36 | 73.0 | 2.84 | 359 | Yes | Metastable $\beta$ | 1777 | 0.0 | 70 | 26146 |
| *Ti-29Nb-13Ta-4Mo* | 16 | 211 | 0.31 | 142.8 | 2.88 | 38 | Yes | Metastable $\beta$ | 1895 | 0.0 | 54 | 34689 |
| *Ti-16Nb-13Ta-1Mo* | 17 | 146 | 0.34 | 111.2 | 2.85 | 112 | Yes | Metastable $\beta$ | 1827 | 0.0 | 64 | 30098 |
| *Ti-16Nb-13Ta-4Mo* | 18 | 121 | 0.36 | 90.3 | 2.84 | 259 | Yes | Metastable $\beta$ | 1801 | 0.0 | 67 | 29918 |
| Example 1 | 1 | 234 | 0.33 | 231 | 2.85 | 136 | Yes | Metastable $\beta$ | 1884 | 1.8 | 53 | 37541 |
| Example 2 | 2 | 212 | 0.33 | 188 | 2.85 | 239 | Yes | Metastable $\beta$ | 1859 | 1.8 | 54 | 42037 |
| Example 3 | 3 | 230 | 0.32 | 218 | 2.85 | 91 | Yes | Metastable $\beta$ | 1875 | 1.8 | 54 | 34251 |
| Example 4 | 4 | 236 | 0.30 | 219 | 2.86 | 87 | Yes | Metastable $\beta$ | 1882 | 1.8 | 53 | 33568 |
| Example 5 | 5 | 245 | 0.31 | 221 | 2.86 | 77 | Yes | Metastable $\beta$ | 1884 | 1.9 | 53 | 31610 |
| Example 6 | 6 | 230 | 0.29 | 210 | 2.86 | 111 | Yes | Metastable $\beta$ | 1875 | 1.8 | 54 | 29315 |
| Example 7 | 7 | 238 | 0.28 | 208 | 2.86 | 105 | Yes | Metastable $\beta$ | 1882 | 1.8 | 53 | 28677 |
| Example 8 | 8 | 247 | 0.27 | 204 | 2.87 | 99 | Yes | Metastable $\beta$ | 1888 | 1.8 | 52 | 28039 |

| | ID | Freezing | CSF | GRF | Bo | Ms | Vital | Weldability | Melting | Al eq. | Ti | Cost |
|---|---|---|---|---|---|---|---|---|---|---|---|---|
| Example 9 | 9 | 248 | 0.25 | 196 | 2.87 | 96 | Yes | Metastable $\beta$ | 1894 | 1.7 | 51 | 27311 |

| Examples | Nb | Mo | Ta | Zr | Sn |
|---|---|---|---|---|---|
| 1 | 18.5 | 4 | 17 | 2.75 | 4.5 |
| 2 | 18.5 | 1.5 | 19 | 2.8 | 4.5 |
| 3 | 20 | 5.25 | 13.5 | 2.75 | 4.5 |
| 4 | 22.5 | 4.75 | 12.5 | 2.5 | 4.5 |
| 5 | 25 | 4.5 | 10.5 | 3 | 4.5 |
| 6 | 27.5 | 3.5 | 8.5 | 2.5 | 4.5 |
| 7 | 30 | 3 | 7.5 | 2.5 | 4.5 |
| 8 | 32.5 | 2.5 | 6.5 | 2.5 | 4.5 |
| 9 | 35 | 2 | 5.5 | 2 | 4.5 |

**Table 5:** Nominal compositions of the new biomedical alloys with optimal manufacturing properties.

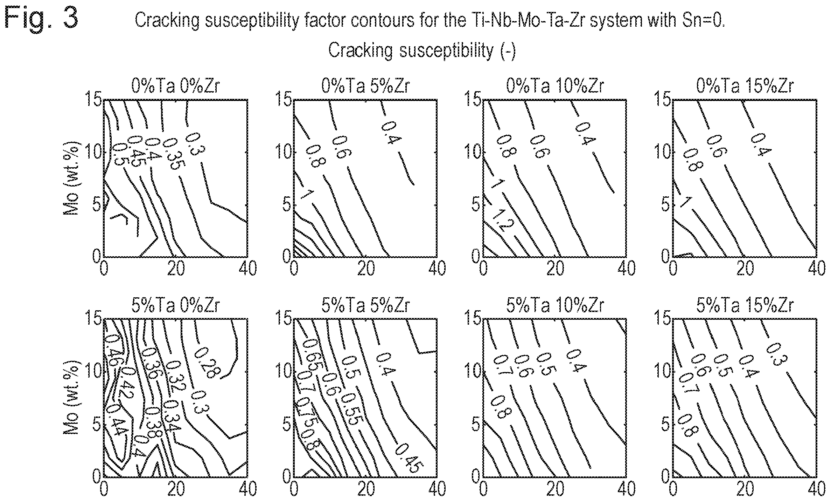

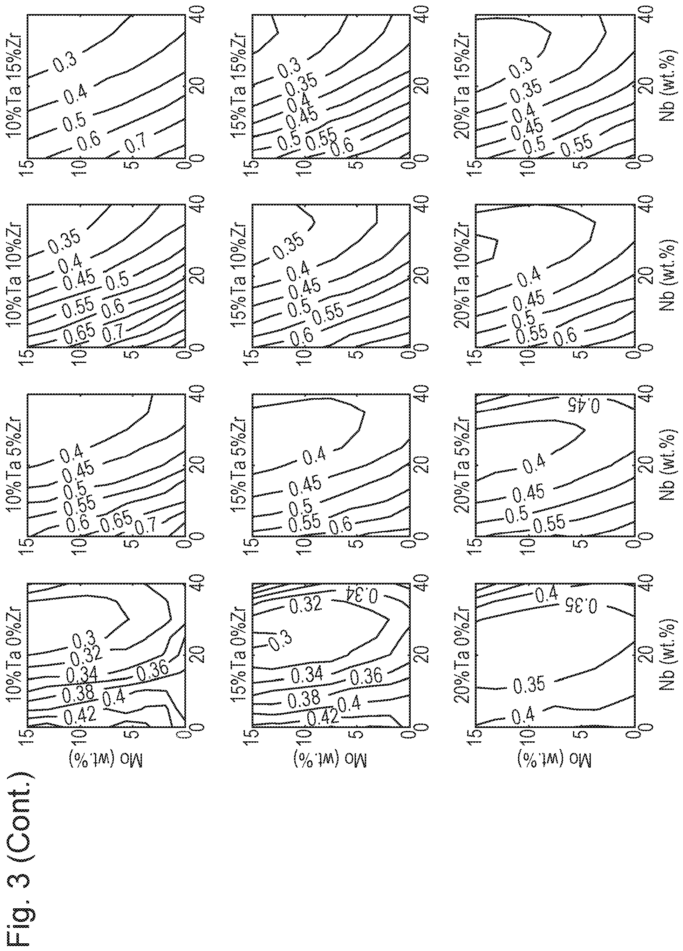

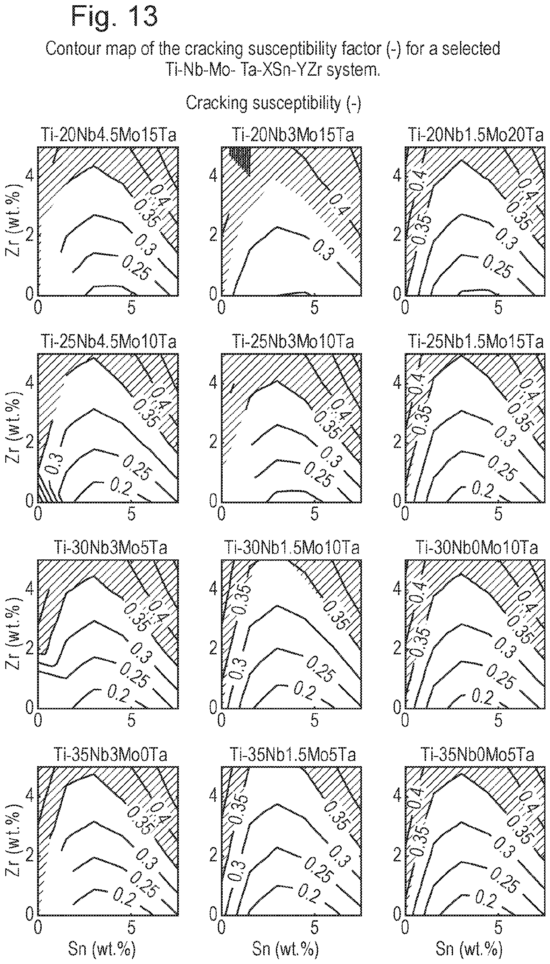

An important characteristic in order to improve the additive manufacturing properties of titanium alloys is a low crack susceptibility factor (CSF), namely the fourth merit index. Figure 3 and 13 illustrate the effects of varying amounts of molybdenum, niobium, tantalum, zirconium and tin on the crack susceptibility factor. The effects of all elements is positive, but the effect of niobium and tin are strongest.

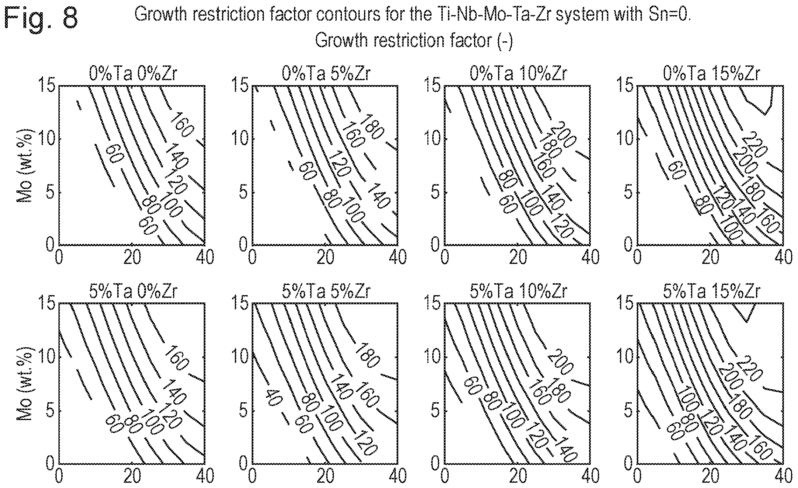

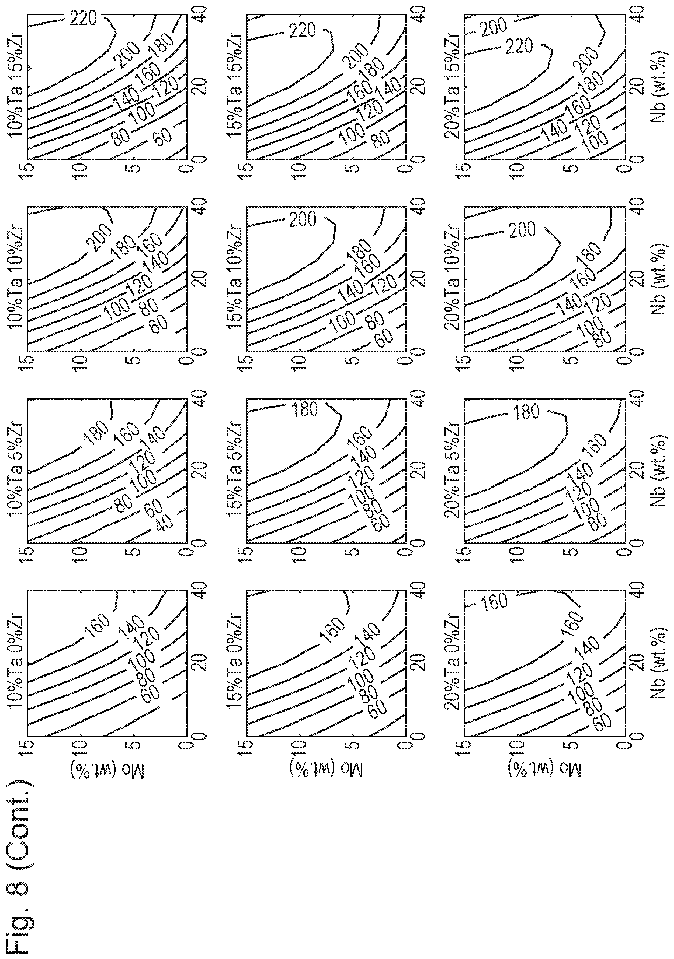

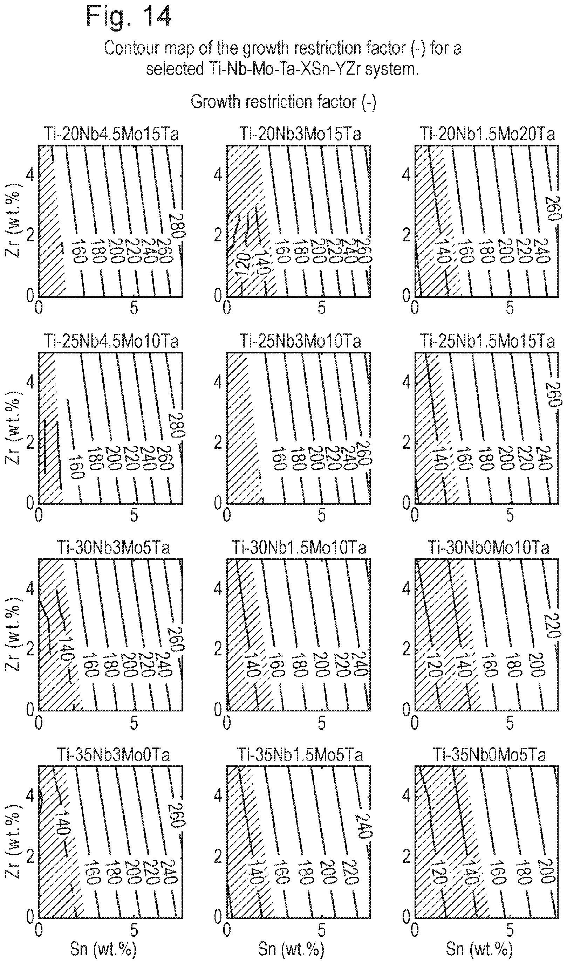

Another important characteristic in order to improve the additive manufacturing properties of titanium alloys is a high growth restriction factor (GRF). Figure 8 and 14 illustrate the effects of varying amounts of molybdenum, niobium, tantalum, zirconium and tin on the growth restriction factor. The effects of all elements is positive, but the effect of niobium, molybdenum and tin is stronger (tin being the strongest of these).

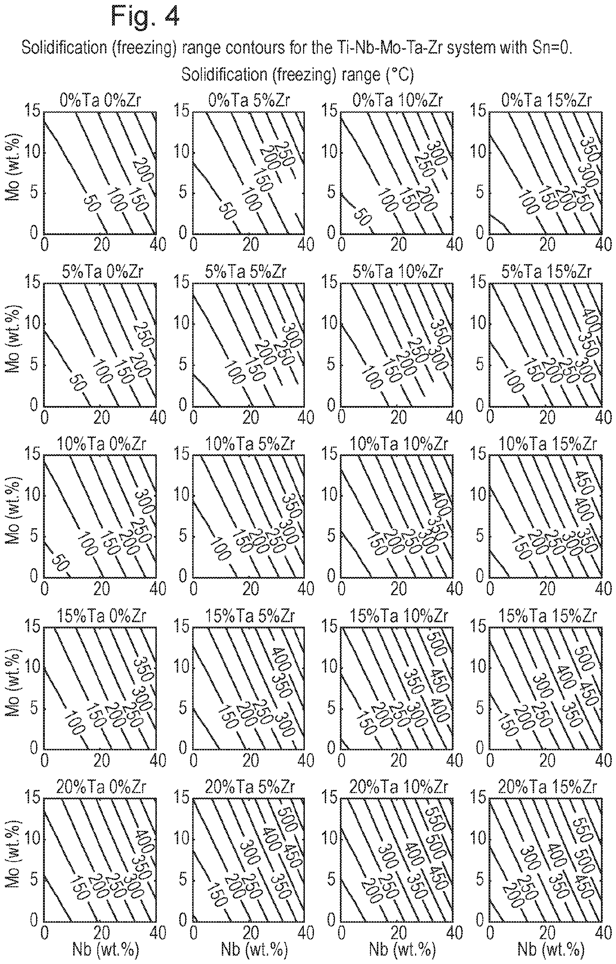

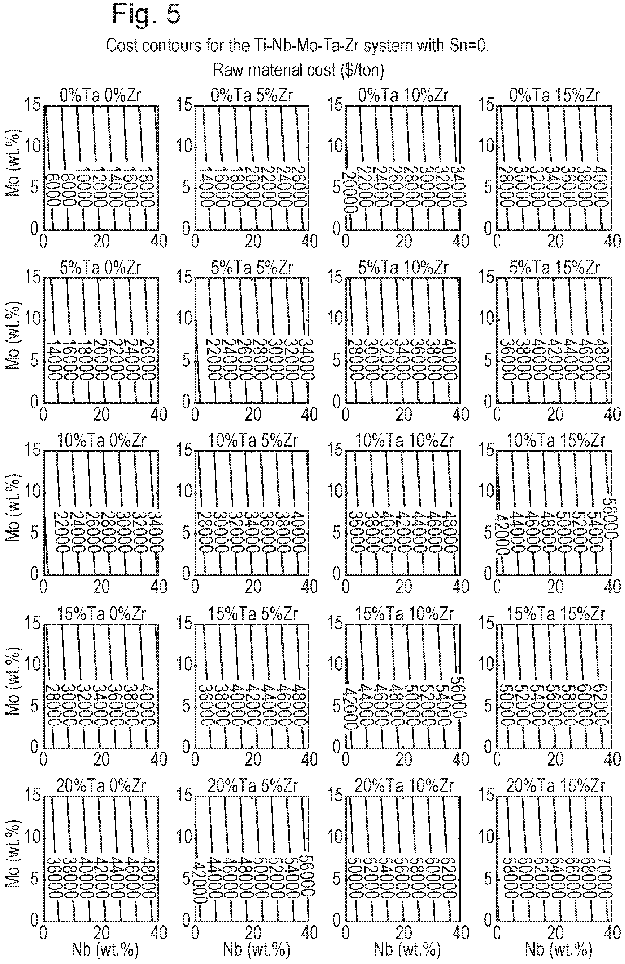

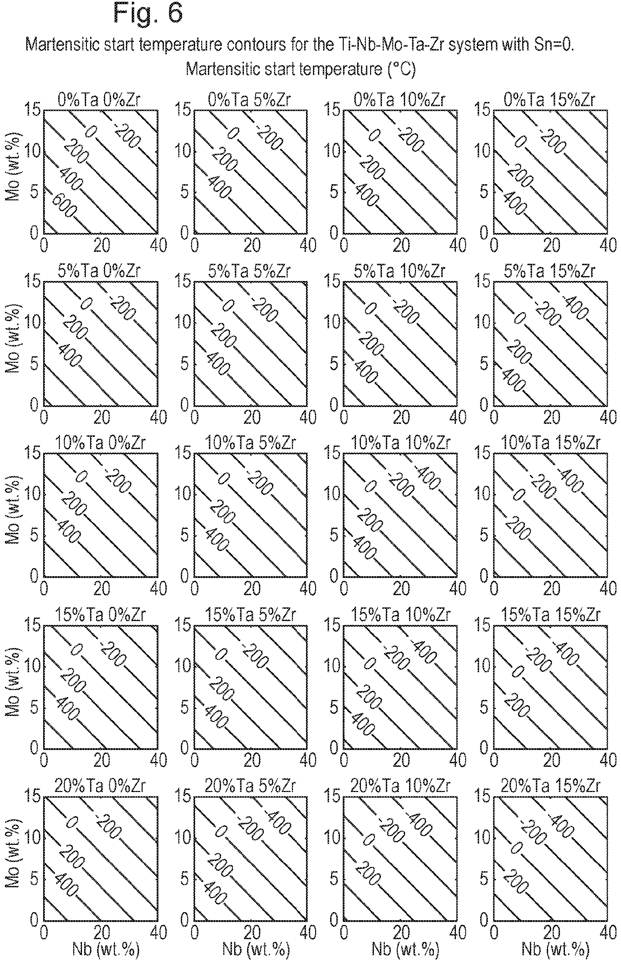

As can be seen, a minimum crack susceptibility factor is achieved with niobium of around 20.0% or more for lower amounts of molybdenum and higher amounts of tantalum or zirconium. Conversely, as can be seen from the contour maps of Figure 4, increased amounts of niobium can deleteriously lead to a greater solidification (freezing) range. This, along with cost (Figure 5), a deleterious effect of decreasing the martensitic start temperature below the desired range (Figure 6) and the desire to keep a minimum amount of titanium as high as possible means that the best balance of properties is achieved when the alloy contains 35.0% or less niobium. An upper limit of 35.0% Nb is set to keep the melting temperature within limits and to allow the addition of other important alloyants which have other positive effects. However, a minimum amount of 15.0% niobium is necessary to achieve a sufficiently low CSF value.

Desirably niobium is added in an amount of at least 17.5 wt.% so as to achieve an even lower CSF and therefore excellent additive manufacturing properties. In a preferred embodiment, niobium is added in an amount of at least 20.0% or even 22.5% yet further to reduce CSF. In an embodiment niobium is limited to 32.5% or less to reduce expense and ease manufacturability of the powder and increase strength and reduce solidification range whilst still maintaining an acceptable level of CSF. In a further preferred embodiment niobium is limited to 30.0 wt.% or less or even 27.5 wt.% or less further reducing the cost of the alloy whilst increasing its strength and ease of manufacture of the starting powder.

Example 2 has a significantly higher martensite start temperature than the other examples. However example 2 uses low niobium and molybdenum levels whilst still achieving an acceptable growth restriction factor. The advantage of such an alloy is that the high levels of Ta and Zr may result in good bio-compatible effects. However, the making of the ingot is the most difficult (Ta very high) and the GRF is not as high as the other examples.

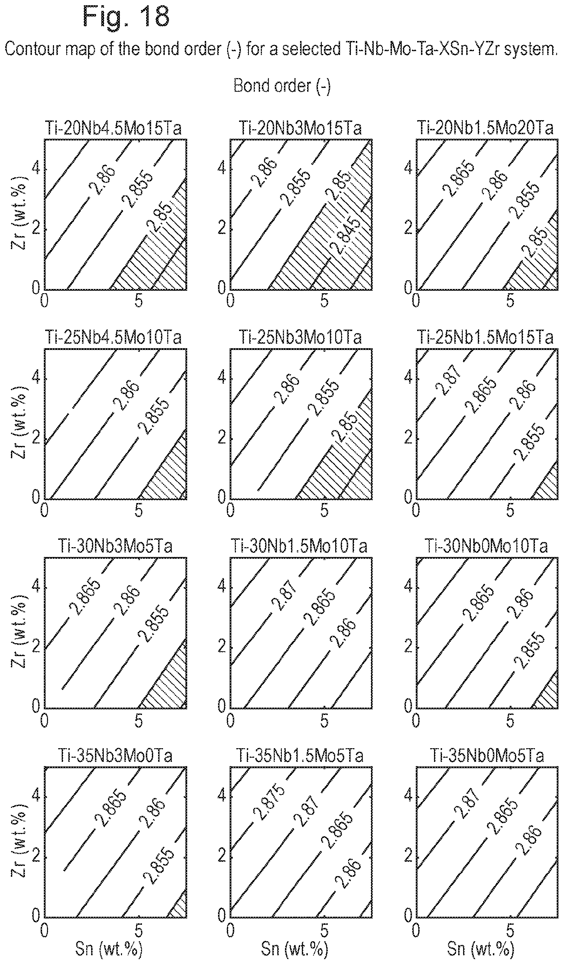

The lowest cracking susceptibility factor is generally achieved for an amount of tin of around 4.5-5.0% (see Figure 13). For amounts of tin higher than 5.0% the CSF begins to deteriorate. At Tin approximately 6.5%, the value of cracking susceptibility tends to reach the restriction limit. Moreover, large amounts of tin tend to lower the bond order of the alloy - Figure 18 shows that for the preferred alloy space, amounts of tin approximately larger than 7.0% tend to produce a value of bond order which is too low. Thus, an upper limit for Tin is imposed at Sn=6.0%. Preferably, tin is present in amounts lower than 5.5% in order to be closer to the optimal CSF value, more preferably lower than 5.0%. In a preferred embodiment, the amount of tin is 4.5 wt.% or less in order to avoid a significant increase of the start martensitic temperature and the amount of $\alpha$-phase present. Moreover, addition of tin can lower the melting temperature of the alloy - this is beneficial. In a preferred embodiment, the amount of tin is 2.0 wt.% or more to add strength and to lower the stiffness but more importantly to increase growth restriction (Figure 14) and to decrease cracking susceptibility. Preferably tin is present in amount of 3.0% or larger. More preferably in an amount of 4.0% or larger.

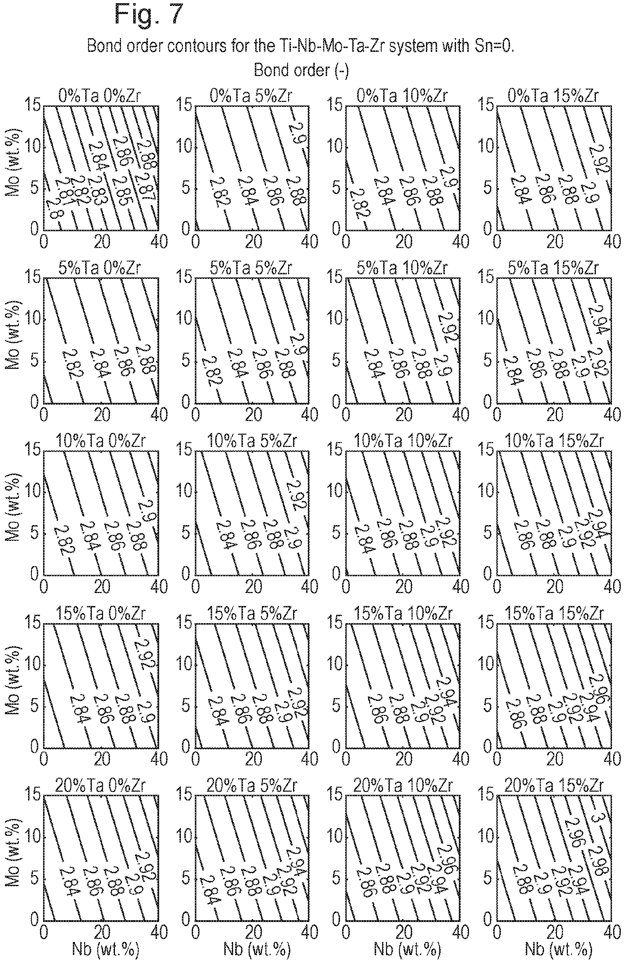

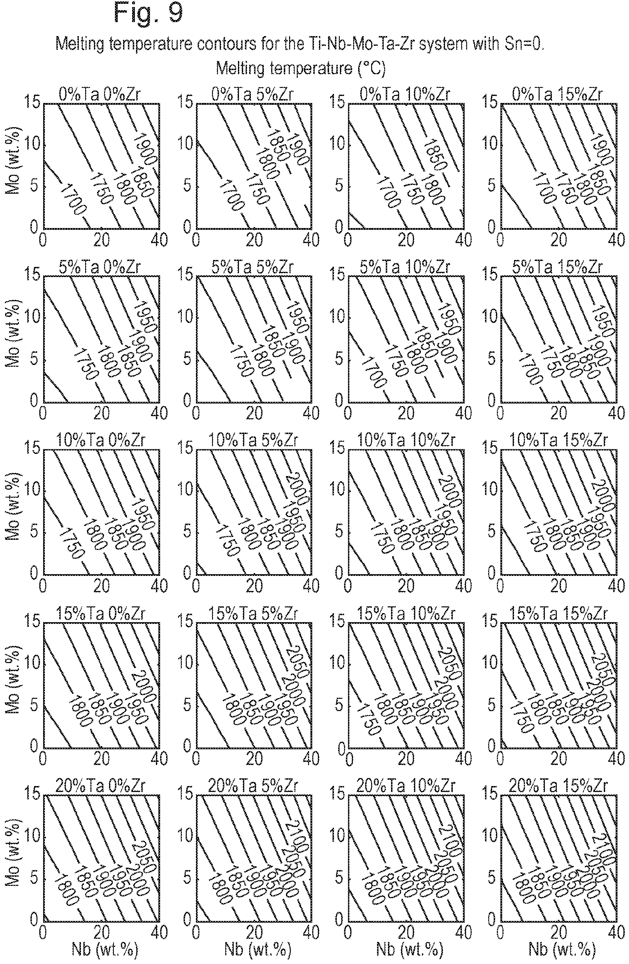

As can be seen from the contour plots of the bond order in Figure 7, tantalum has a strong influence on advantageously increasing the bond order. This promotes cell growth and indicates decreased toxicity. On the other hand, tantalum is deleterious in terms of cost of the alloy (Figure 5) and in terms of reduced manufacturability due to increasing of the solidification range (Figure 4) and in terms of reduced strength due to lowering of the martensitic start temperature (Figure 6), although this deleterious effect on the martensitic start temperature is not so strong. Tantalum also increases the melting temperature of the alloy (Figure 9). An upper limit of 20.0% Ta is set in order to maintain a sufficiently low melting point. Desirably the maximum amount of tantalum is 17.5% or less (for instance preferably 15.0% or less or 12.5% or less tantalum) as this can help in the manufacturability of the atomised alloy (because this allows a higher amount of titanium so that the possibility of segregation during atomisation is reduced) and reduces the raw cost of the alloy significantly.

To achieve a bond order which is comparable with the best performing existing alloys shown in Table 3, the amount of tantalum is preferably 5.0% or more, more preferably 7.5% or more. A bonding order even greater can be achieved by increasing the minimum amount of tantalum to 10.0 wt.% or more or even 12.5% or more. However, tantalum may be absent, depending upon the combination of properties desired.

Molybdenum has a strong effect on reducing the martensitic start temperature and thus β-stabilising the alloy (Figure 6) while keeping the content of pure titanium high. Molybdenum is also important in increasing the growth restriction factor (Figure 8) and to some extent reducing the cracking susceptibility factor (Figure 3). However, molybdenum has the effect of increasing the overall solidification range (Figure 4). Nonetheless, molybdenum is a known grain refiner and a strong β stabiliser, thus, it allows an alloy with a high titanium content to be produced (thereby increasing the ease of powder processing and its chemical homogeneity). Along with niobium and tantalum, molybdenum undesirably increases the melting temperature (Figure 9). Therefore, it has been found that a suitable level of molybdenum is 7.5 wt.% or less. Because of its beneficial effect on GRF, molybdenum is preferably present in an amount of at least 1.5 wt.% or more. Desirably molybdenum is present in amount of 7.0 wt.% or less to reduce its effect on the increase on solidification range and to assure that the martensite start temperature range remains within the desired range. Levels of molybdenum of 6.0% or less or even 5.0% or less are even more desirable for the same reason. On the other hand, an amount of molybdenum of 2.5 wt% or more, preferably 3.5 wt.% or more is advantageous as this ensures the GRF remaining high without needing further to increase the amount of niobium.

The contour plots of Figures 3-6 show that the effect of zirconium is not as strong as the other alloying elements except in a deleterious way relating to the cost (Figure 5). Zirconium has little effect on the melting temperature (Figure 9) or on the martensitic start temperature (Figure 6) and its effect on the CSF (Figure 3) seems less strong than Nb, Mo and Ta. However, Zr is known to substitute for Ti while adding bio-compatibility. Moreover, Zr is known to lower the stiffness (it substantially increases bonding order - see Figure 7) and adding strength by solidsolutioning and grain refinement (Zr increases GRF - see Figure 8). However, large amounts of Zr are shown to have a negative effect on the cracking susceptibility (see Figure 3). With the above in mind, small amounts of zirconium may be added, particularly because of its known biocompatibility (see Figure 1) and its strengthening effect. However, due to the desire of keeping a low cracking susceptibility factor, the suitable maximum amount of zirconium is preferably 6.0wt% or less, even 5.5% or less. However, up to 7.0% or less is permitted, particularly where a strong alloy is desired at the expense of additive manufacturability. Limiting the amount of Zr also ensures a high Ti content and a low solidification range (Figure 4). Moreover, Zr is restricted to small amounts because its effect on the CSF is not as beneficial as Nb, Ta and Mo. In a preferred embodiment the alloy consists of 4.5% or less zirconium, preferably 4.0% or less or even 3.5% or less zirconium to ensure a low cracking susceptibility. In a preferred embodiment, a minimum amount of 1.0% or more is desirable in order to increase the bond order, to increase the growth restriction factor and to add strength. More preferably 1.5% or more or 2.0% or more further to increase the bond order, to further increase the growth restriction factor and to add strength.

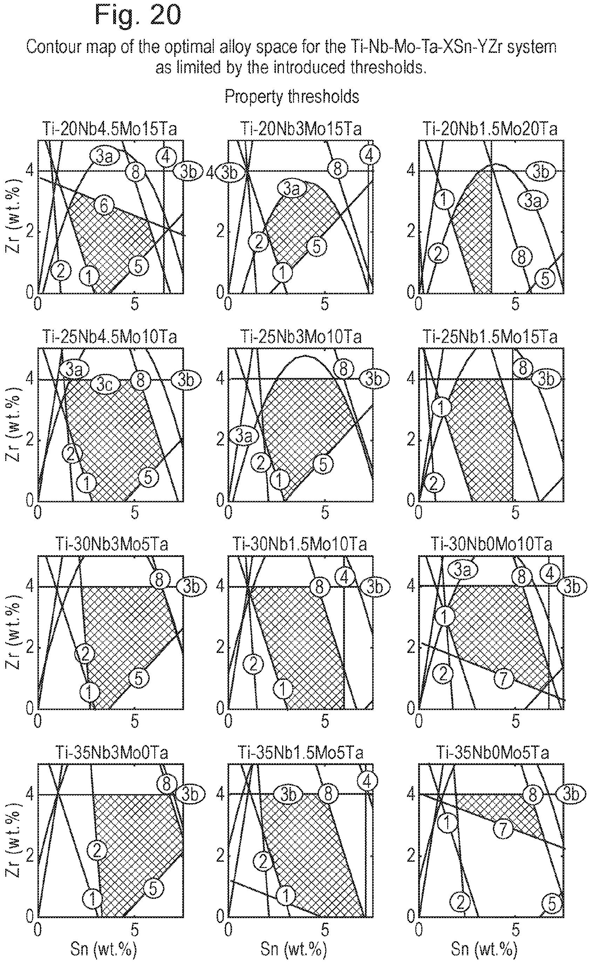

Figure 20 is a summary of the preferable requirements of the alloy together with a series of linearised restrictions which were derived to isolate the optimal alloy space.

Figure 20 has plots for compositions falling within the preferred range and is simply used to illustrate further restrictions on the alloy which result in the best combination of the first-sixth merit indices.

Regression analysis was performed on the results of the calculations of the merit indices to determine the following seven relationships which are valid within the elemental range of the alloy (Nb 15-35, Mo 0-7.5, Ta 0-20.0, Zr 0-7.0 and Sn 0-6.0). As a main requirement for the alloy a growth restriction factor (GRF) of at least 150 was chosen. This is greater than the GRF achieved by any of the prior art alloys in table 4. As described above in relation to the fifth merit index, a higher growth restriction factor is beneficial for additive manufacturing. From Figure 8 it becomes clear that a region of the alloy space investigated results in alloys with a growth restriction factor greater than those of the prior art (as shown in Table 4). Analysis shows that within the elemental range of the alloy space, a growth restriction factor of greater than 150 can be achieved if the following equation (equation 2) is satisfied:

$$0.0175Nb + 0.0183Mo + 0.03Ta + 0.0116Zr + 0.1Sn > 1.0, \text{ preferably} > 1.1, \text{ more preferably} > 1.2$$

where Nb, Mo, Ta, Zr and Sn represent the amounts of niobium, molybdenum, tantalum, zirconium and tin in weight percent respectively. Preferably the growth restriction factor is even greater meaning that the sum on the left hand side of equation 2 is greater than 1.1, even more preferably greater than 1.2, as shown above. As shown in Table 5, all the example alloys have a growth restriction factor of greater than 180 and the above sum is at least 1.28. The best performing alloys in this respect have a growth restriction factor of greater than 200 and this is also preferred as the greater the growth restriction factor the greater the additive manufacturability. This equation is marked as equation 2 in Figure 20.

Line 1 in Figure 20 represents the following equation

$$1.0 \leq \text{Al wt. \%} + \frac{1}{3}\text{Sn wt. \%} + \frac{1}{6}\text{Zr wt. \%} \leq 2.5$$

Meeting this criterion ensures that the strength benefits of having enough aluminium equivalent elements is met - because aluminium itself is restricted due to low bio-compatibility. Because of the low levels of allowed aluminium, aluminium can be excluded from this equation.

The fourth merit index (crack susceptibility factor) is represented by two equations marked as 3a and 3b in Figure 20. As can be seen in Figure 3, in the design space considered a minimum crack susceptibility factor is present in the alloy design space and the relationship is not linear. Therefore two linear equations are used to model that desired space. These equations are $0.0375Nb + 0.033Mo + 0.0167Ta + 0.05Zr + 0.267 - (0.13Sn - 0.516)^2 > 1.0$ which is shown as 3a in Figure 20 and Zr>4 which is shown as equation 3b in Figure 20 These equations are generated with the requirement that the crack susceptibility factor is 0.33 or less which can be seen to be lower than the crack susceptibility factor of most of the prior art alloys shown in Table 5. Preferably, the crack susceptibility value is lower than 0.3 or even 0.28. For those cases, one wants to achieve values of Eq. 3a approximately higher than 1.17 but values of equation 3a of higher than 1.1 also have very low crack susceptibility factor and are only slightly less preferred. As shown in Table 5, the example alloys 6 to 9 have a cracking susceptibility lower than 0.3 - thus better than all the prior art alloys. When equation 3a is used, these example alloys have a value greater than 1.17 - this is preferred as the greater the value of equation 3a, the better the resistance to cracking during the additive manufacturing process.

The sixth merit index is modelled by the following equation (equation 4) $0.0178Nb + 0.0143Mo + 0.0243Ta +0.0285Sn < 1.0$ which is shown as line 4 in Figure 20. Alloys meeting this equation have a melting point of below 1900°C.

The first merit index is desirably an alloy with a bonding order of greater than 2.85. This requirement is met by an alloy satisfying the following equation (equation 5) $0.0298Nb + 0.0272Mo + 0.0246Ta + 0.0376Zr + 0.0259Sn > 1.0$ where Zr, Nb, Mo, Ta and Sn represent the amounts of zirconium, niobium, molybdenum, tantalum and tin respectively in wt%. Such an alloy has a bonding order comparable to that of prior art alloys as shown in Table 4. This equation is shown as line 5 in Figure 20.These alloys have high enough bond order for improved bio-compatibility and low elastic modulus. If the sum of equation 5 is greater than 1.1, the bond order is even higher which is desirable.

The second merit index, namely the susceptibility to martensitic phase is already described above with respect to the equation for Ms. The same line is plotted in Figure 20 for the components illustrated in Figure 20. Lines 6 and 7 marking the maximum and minimum desired martensitic start temperature (250°C and 75°C) are plotted in Figure 20 and the equation is as follows:

$$75 > 883\text{-}150\text{Fe-}49\text{Mo} - 17\text{Nb} - 12\text{Ta} - 7\text{Zr} - 3\text{Sn} < 250.$$

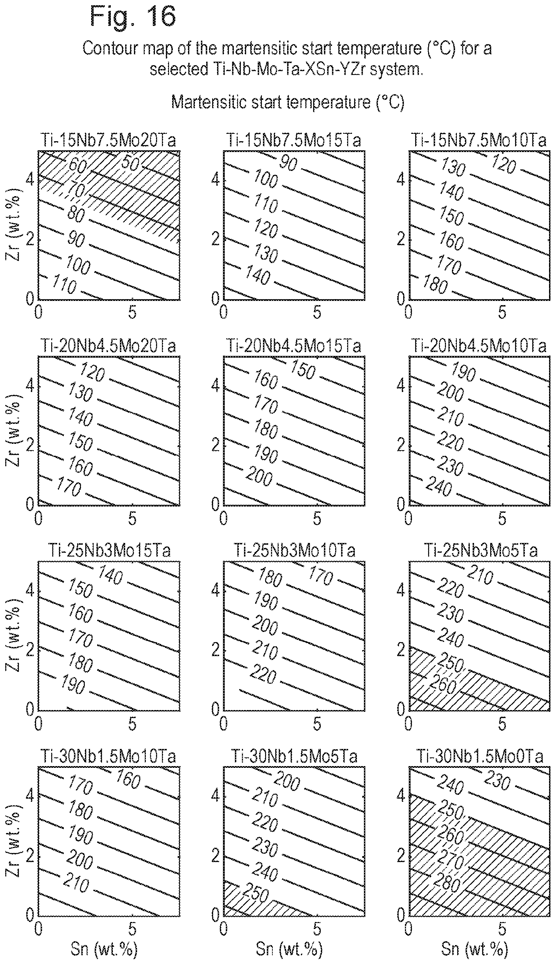

The desirable maximum allowable martensite start levels of 225°C and 220°C are not plotted in figure 20, but from figure 16 the effect of such preferred restrictions on allowable amounts of alloyants can be seen.

The third merit index, namely the freezing range, is also plotted with a desirable range of less than 250°C is plotted as line 8 in Figure 20 (the upper end of the range; all the alloys in figure 20 have a freezing range above the lower end of the range. This follows the following equation (equation 8):

$$2.5 > 0.042\text{Nb} + 0.06\text{Mo} + 0.05\text{Ta} + 0.03\text{Zr} + 0.1\text{Sn} > 1.0.$$

This equation 8 isolates those alloys which have an optimal solidification range - high enough to allow optimal layer adhesion during additive manufacturing and low enough so that the risk of hot tearing is kept low.

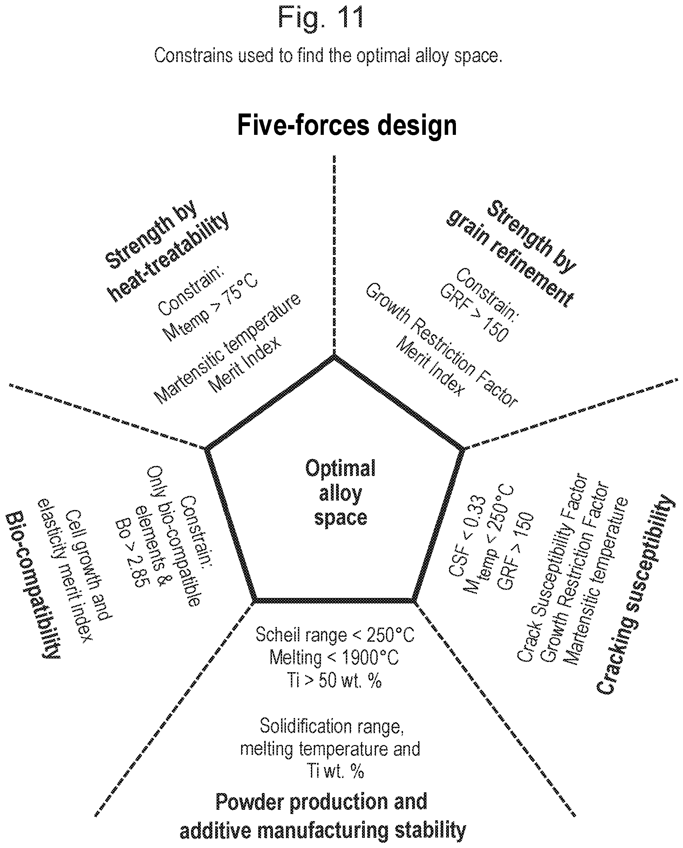

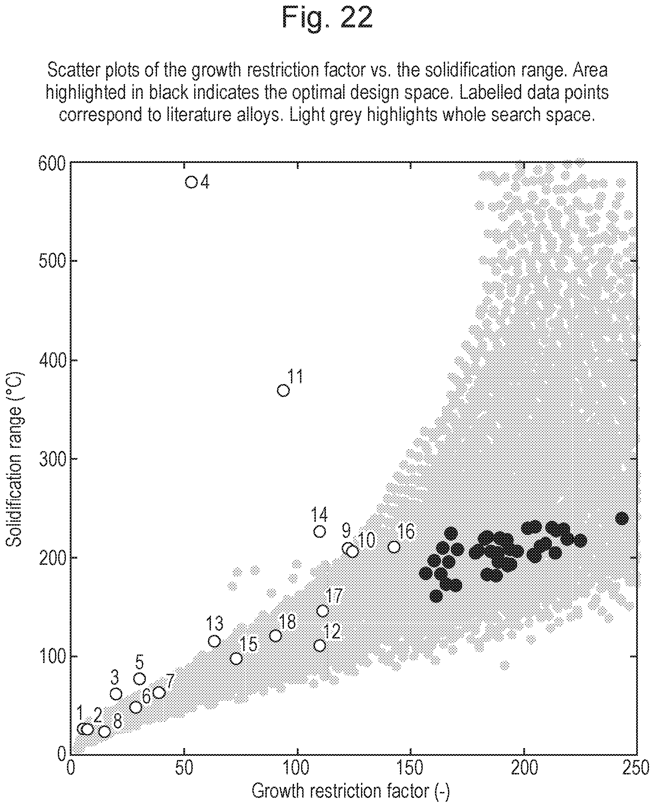

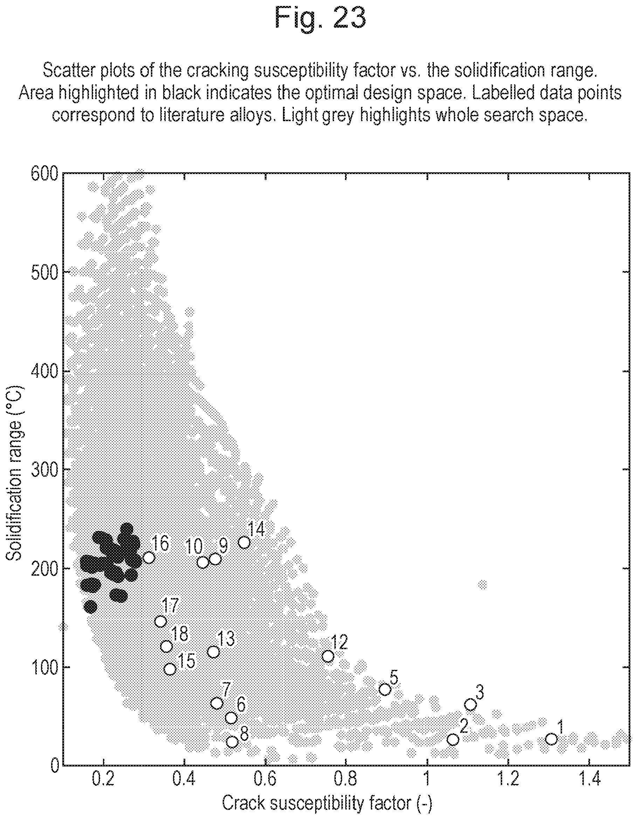

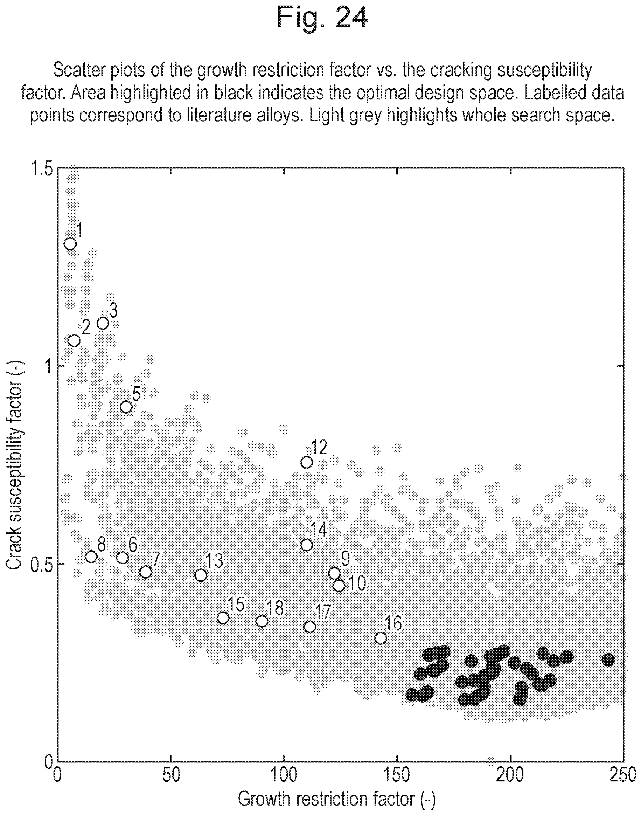

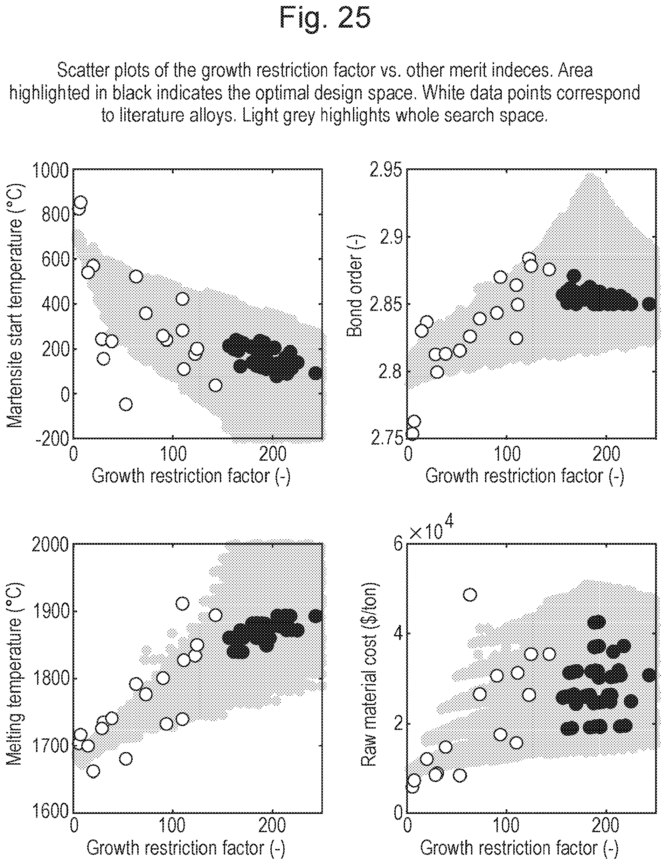

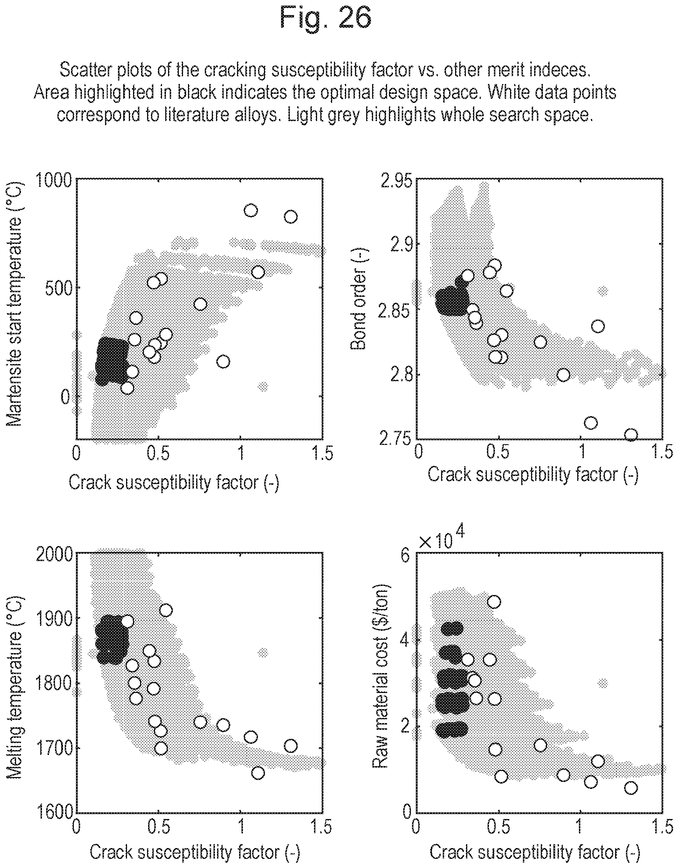

Figures 22-26 illustrate the range of merit indices of alloys in the range in which the model was run (Table 2) with the properties of existing alloys numbered and plotted on the same graphs as well as areas of the inventive range plotted (as restricted by Figure 11). As can be seen, the alloys of the present invention have improved properties over the otherwise most suitable existing alloys.

The amounts of silicon and boron are not arrived at from thermodynamic calculations but instead are added from knowledge that they will increase strength and creep resistance and enhance ductility of the alloy. As such, they are allowable up to 0.2 wt% boron and 1.0 wt% silicon.

## Claims

1. A titanium-based alloy composition consisting in weight percent, of:
   between 15.0 and 35.0 % niobium, between 0.0 and 7.5 % molybdenum, between 0.0 and 20.0% tantalum, between 0 and 7.0% zirconium, between 0 and 6.0% tin, between 0.0 and 2.0% hafnium, between 0.0 and 0.5 % aluminium, between 0.0 and 0.5 % vanadium, between 0.0 and 0.5 % iron, between 0.0 and 0.5 % chromium, between 0.0 and 0.5% cobalt, between 0.0 and 0.5 % nickel, between 0.0 and 1.0% silicon, between 0.0 and 0.2% boron, between 0.0 and 0.5% calcium, between 0.0 and 0.5% carbon, between 0.0 and 0.5% manganese, between 0.0 and 0.5% gold, between 0.0 and 0.5% silver, between 0.0 and 0.5% oxygen, between 0.0 and 0.5% hydrogen, between 0.0 and 0.5% nitrogen, between 0.0 and 0.5% palladium, between 0.0 and 0.5% lanthanum, the balance being titanium and incidental impurities, wherein the composition satisfies the following equation:

$$0.0175\text{Nb} + 0.0183\text{Mo} + 0.03\text{Ta} + 0.0116\text{Zr} + 0.1\text{Sn} > 1.0$$

   where Nb, Mo, Ta, Zr and Sn represent the amounts of niobium, molybdenum, tantalum, zirconium and tin in wt% respectively.

2. The titanium-based alloy composition of claim 1, consisting of 7.0% or less molybdenum, preferably 6.0% or less molybdenum, more preferably 5.0 or less molybdenum.

3. The titanium-based alloy composition of claim 1 or 2, consisting of 1.5% or more molybdenum, preferably of 2.5% or more molybdenum.

4. The titanium-based alloy composition of claim 1, 2 or 3 , which satisfies the following equation $0.0375\text{Nb} + 0.033\text{Mo} + 0.0167\text{Ta} + 0.05\text{Zr} + 0.267 - (0.13\text{Sn} - 0.516)^2 > 1.0$, preferably > 1.1 where Zr, Sn, Mo, Ta and Nb represent the amounts of zirconium, tin, molybdenum, tantalum and niobium in wt % respectively.

5. The titanium-based alloy composition of any of claims 1 to 4, either (i) consisting of 5.5% or less tin, preferably 5.0% or less tin, more preferably 4.75% or less tin and/or (ii) consisting of 2.0% or more tin, preferably 3.0% or more tin, more preferably 4.0% or more tin.

6. The titanium-based alloy composition of any of claims 1 to 5, either (i) consisting of 6.0% or less zirconium, preferably 5.5% or less zirconium, more preferably 4.5% or less zirconium, even more preferably 4.0% or less zirconium, most preferably 3.5% or less zirconium and/or (ii) consisting of 1.0% or more zirconium, preferably 1.5% or more zirconium, more preferably 2.0% or more zirconium

7. The titanium-based alloy composition of any of claims 1 to 6, which satisfies the flowing relationship

$$1.0 \ \le \text{Al wt.} \% + \frac{1}{3}\text{Sn wt.} \% + \frac{1}{6}\text{Zr wt.} \% \ \le 2.5$$

   in which Al, Sn and Zr represent the amounts of aluminium, tin and zirconium in wt% respectively.

8. The titanium-based alloy composition of any of claims 1 to 7, either (i) consisting of 17.5% or more niobium, preferably 20.0% or more niobium, more preferably 22.5% or more niobium and/or (ii) consisting of 32.5% or less niobium, preferably 30.0% or less niobium, more preferably 27.5% or less niobium.

9. The titanium-based alloy composition of any of claims 1 to 8, either (i) consisting of 17.5% or less tantalum, preferably 15.0% or less tantalum, more preferably 12.5% or less tantalum and/or (ii) consisting of 5.0% or more tantalum, preferably 7.5% or more tantalum, more preferably 10.0% or more tantalum, most preferably 12.5% or more tantalum.

10. The titanium-based alloy composition of any of claims 1 to 9, either (i) which satisfies the flowing equation

$$250 \geq 883 - 150\,Fe\,wt.\% - 96\,Cr\,wt.\% - 49\,Mo\,wt.\% - 37\,V\,wt.\% - 17\,Nb\,wt.\% - 12\,Ta\,wt.\% - 7\,Zr\,wt.\% - 3\,Sn\,wt.\% + 15\,Al\,wt.\%,$$

preferably

$$225 \geq 883 - 150\,Fe\,wt.\% - 96\,Cr\,wt.\% - 49\,Mo\,wt.\% - 37\,V\,wt.\% - 17\,Nb\,wt.\% - 12\,Ta\,wt.\% - 7\,Zr\,wt.\% - 3\,Sn\,wt.\% + 15\,Al\,wt.\%,$$

more preferably

$$200 \geq 883 - 150\,Fe\,wt.\% - 96\,Cr\,wt.\% - 49\,Mo\,wt.\% - 37\,V\,wt.\% - 17\,Nb\,wt.\% - 12\,Ta\,wt.\% - 7\,Zr\,wt.\% - 3\,Sn\,wt.\% + 15\,Al\,wt.\%$$

in which Fe, Cr, Mo, V, Nb, Ta, Zr, Sn and Al represent the amounts of iron, chromium, molybdenum, vanadium, niobium, tantalum, zirconium, tin and aluminium in wt% respectively and/or which satisfies the flowing equation

$$75 \leq 883 - 150\,Fe\,wt.\% - 96\,Cr\,wt.\% - 49\,Mo\,wt.\% - 37\,V\,wt.\% - 17\,Nb\,wt.\% - 12\,Ta\,wt.\% - 7\,Zr\,wt.\% - 3\,Sn\,wt.\% + 15\,Al\,wt.\% \leq 250$$

in which Fe, Cr, Mo, V, Nb, Ta, Zr, Sn and Al represent the amounts of iron, chromium, molybdenum, vanadium, niobium, tantalum, zirconium, tin and aluminium in wt% respectively.

**11.** The titanium-based alloy composition of any of claims 1 to 10, which satisfies the following equation

$$0.0178Nb + 0.0143Mo + 0.0243Ta + 0.0285Sn < 1.0$$

in which Mo, Nb, Ta, and Sn represent the amounts of molybdenum, niobium, tantalum and tin in wt% respectively.

**12.** The titanium-based alloy composition of any of claims 1 to 11, which satisfies the following equation

$$0.0298Nb + 0.0272Mo + 0.0246Ta + 0.0376Zr + 0.0259Sn > 1.0,$$

preferably

$$0.0298Nb + 0.0272Mo + 0.0246Ta + 0.0376Zr + 0.0259Sn > 1.1$$

where Zr, Nb, Mo, Ta and Sn represent the amounts of zirconium niobium, molybdenum, tantalum and tin respectively in wt%.

**13.** The titanium-based alloy composition of any of claims 1 to 12, either (i) which satisfies the following equation

2.5 > 0.042Nb + 0.06Mo + 0.05Ta + 0.03Zr +0.1Sn in which Mo, Nb, Ta, Zr and Sn represent the amounts of molybdenum, niobium, tantalum, zirconium and tin in wt% respectively;
and/or (ii) which satisfies the following equation

$$0.042Nb + 0.06Mo + 0.05Ta + 0.03Zr + 0.1Sn > 1.0$$

in which Mo, Nb, Ta, Zr and Sn represent the amounts of molybdenum, niobium, tantalum, zirconium and tin in wt% respectively.

**14.** An implant or prosthetic device made of the titanium-based alloy of any of claims 1-13.

**15.** A method of additive manufacturing an article which uses the titanium-based alloy of any of claims 1-13, preferably wherein the article is an implant or prosthetic device.

**Patentansprüche**

**1.** Legierungszusammensetzung auf Titanbasis bestehend in Gewichtsprozent aus:
zwischen 15,0 und 35,0 % Niob, zwischen 0,0 und 7,5 % Molybdän, zwischen 0,0 und 20,0 % Tantal, zwischen 0 und 7,0 % Zirconium, zwischen 0 und 6,0 % Zinn, zwischen 0,0 und 2,0 % Hafnium, zwischen 0,0 und 0,5 % Aluminium, zwischen 0,0 und 0,5 % Vanadium, zwischen 0,0 und 0,5 % Eisen, zwischen 0,0 und 0,5 % Chrom, zwischen 0,0 und 0,5 % Cobalt, zwischen 0,0 und 0,5 % Nickel, zwischen 0,0 und 1,0 % Silicium, zwischen 0,0 und 0,2 % Bor, zwischen 0,0 und 0,5 % Calcium, zwischen 0,0 und 0,5 % Kohlenstoff, zwischen 0,0 und 0,5 % Mangan, zwischen 0,0 und 0,5 % Gold, zwischen 0,0 und 0,5 % Silber, zwischen 0,0 und 0,5 % Sauerstoff, zwischen 0,0 und 0,5 % Wasserstoff, zwischen 0,0 und 0,5 % Stickstoff, zwischen 0,0 und 0,5 % Palladium, zwischen 0,0 und 0,5 % Lanthan, wobei der Rest Titan und zufällige Verunreinigungen sind, wobei die Zusammensetzung die folgende Gleichung erfüllt:

$$0,0175 \text{ Nb} + 0,0183 \text{ Mo} + 0,03 \text{ Ta} + 0,0116 \text{ Zr} + 0,1 \text{ Sn} > 1,0,$$

wobei Nb, Mo, Ta, Zr und Sn die Mengen an Niob, Molybdän, Tantal, Zirconium bzw. Zinn in Gew.-% darstellen.

**2.** Legierungszusammensetzung auf Titanbasis nach Anspruch 1, die aus 7,0 % oder weniger Molybdän, bevorzugt 6,0 % oder weniger Molybdän, besonders bevorzugt 5,0 oder weniger Molybdän besteht.

**3.** Legierungszusammensetzung auf Titanbasis nach Anspruch 1 oder 2, die aus 1,5 % oder mehr Molybdän, bevorzugt aus 2,5 % oder mehr Molybdän besteht.

**4.** Legierungszusammensetzung auf Titanbasis nach Anspruch 1, 2 oder 3, die die folgende Gleichung erfüllt: $0,0375 \text{ Nb} + 0,033 \text{ Mo} + 0,0167 \text{ Ta} + 0,05 \text{ Zr} + 0,267 - (0,13 \text{ Sn} - 0,516)^2 > 1,0$, bevorzugt $> 1,1$, wobei Zr, Sn, Mo, Ta und Nb die Mengen an Zirconium, Zinn, Molybdän, Tantal bzw. Niob in Gew.-% darstellen.

**5.** Legierungszusammensetzung auf Titanbasis nach einem der Ansprüche 1 bis 4, die entweder (i) aus 5,5 % oder weniger Zinn, bevorzugt 5,0 % oder weniger Zinn, besonders bevorzugt 4,75 % oder weniger Zinn besteht und/oder (ii) aus 2,0 % oder mehr Zinn, bevorzugt 3,0 % oder mehr Zinn, besonders bevorzugt 4,0 % oder mehr Zinn besteht.

**6.** Legierungszusammensetzung auf Titanbasis nach einem der Ansprüche 1 bis 5, die entweder (i) aus 6,0 % oder weniger Zirconium, bevorzugt 5,5 % oder weniger Zirconium, besonders bevorzugt 4,5 % oder weniger Zirconium, ganz besonders bevorzugt 4,0 % oder weniger Zirconium, am meisten bevorzugt 3,5 % oder weniger Zirconium besteht und/oder (ii) aus 1,0 % oder mehr Zirconium, bevorzugt 1,5 % oder mehr Zirconium, besonders bevorzugt 2,0 % oder mehr Zirconium besteht.

**7.** Legierungszusammensetzung auf Titanbasis nach einem der Ansprüche 1 bis 6, die die Flussbeziehung

$$1,0 \leq \text{Al Gew.-\%} + 1/3 \text{ Sn Gew.-\%} + 1/6 \text{ Zr Gew.-\%} \leq 2,5$$

erfüllt,
in der Al, Sn und Zr die Mengen an Aluminium, Zinn bzw. Zirconium in Gew.-% darstellen.

**8.** Legierungszusammensetzung auf Titanbasis nach einem der Ansprüche 1 bis 7, die entweder (i) aus 17,5 % oder mehr Niob, bevorzugt 20,0 % oder mehr Niob, besonders bevorzugt 22,5 % oder mehr Niob besteht und/oder (ii) aus 32,5 % oder weniger Niob, bevorzugt 30,0 % oder weniger Niob, besonders bevorzugt 27,5 % oder weniger Niob besteht.

9.  Legierungszusammensetzung auf Titanbasis nach einem der Ansprüche 1 bis 8, die entweder (i) aus 17,5 % oder weniger Tantal, bevorzugt 15,0 % oder weniger Tantal, besonders bevorzugt 12,5 % oder weniger Tantal besteht und/oder (ii) aus 5,0 % oder mehr Tantal, bevorzugt 7,5 % oder mehr Tantal, besonders bevorzugt 10,0 % oder mehr Tantal, am meisten bevorzugt 12,5 % oder mehr Tantal besteht.

10. Legierungszusammensetzung auf Titanbasis nach einem der Ansprüche 1 bis 9, (i) die entweder die Flussgleichung

$$250 \geq 883 - 150\ \text{Fe Gew.-\%} - 96\ \text{Cr Gew.-\%} - 49\ \text{Mo Gew.-\%} - 37\ \text{V Gew.-\%} - 17\ \text{Nb Gew.-\%} - 12\ \text{Ta Gew.-\%} - 7\ \text{Zr Gew.-\%} - 3\ \text{Sn Gew.-\%} + 15\ \text{Al Gew.-\%},$$

bevorzugt

$$225 \geq 883 - 150\ \text{Fe Gew.-\%} - 96\ \text{Cr Gew.-\%} - 49\ \text{Mo Gew.-\%} - 37\ \text{V Gew.-\%} - 17\ \text{Nb Gew.-\%} - 12\ \text{Ta Gew.-\%} - 7\ \text{Zr Gew.-\%} - 3\ \text{Sn Gew.-\%} + 15\ \text{Al Gew.-\%},$$

besonders bevorzugt

$$200 \geq 883 - 150\ \text{Fe Gew.-\%} - 96\ \text{Cr Gew.-\%} - 49\ \text{Mo Gew.-\%} - 37\ \text{V Gew.-\%} - 17\ \text{Nb Gew.-\%} - 12\ \text{Ta Gew.-\%} - 7\ \text{Zr Gew.-\%} - 3\ \text{Sn Gew.-\%} + 15\ \text{Al Gew.-\%}$$

erfüllt,
in der Fe, Cr, Mo, V, Nb, Ta, Zr, Sn und Al die Mengen an Eisen, Chrom, Molybdän, Vanadium, Niob, Tantal, Zirconium, Zinn bzw. Aluminium in Gew.-% darstellen, und/oder die die Flussgleichung

$$75 \leq 883 - 150\ \text{Fe Gew.-\%} - 96\ \text{Cr Gew.-\%} - 49\ \text{Mo Gew.-\%} - 37\ \text{V Gew.-\%} - 17\ \text{Nb Gew.-\%} - 12\ \text{Ta Gew.-\%} - 7\ \text{Zr Gew.-\%} - 3\ \text{Sn Gew.-\%} + 15\ \text{Al Gew.-\%} \leq 250$$

erfüllt,
in der Fe, Cr, Mo, V, Nb, Ta, Zr, Sn und Al die Mengen an Eisen, Chrom, Molybdän, Vanadium, Niob, Tantal, Zirconium, Zinn bzw. Aluminium in Gew.-% darstellen.

11. Legierungszusammensetzung auf Titanbasis nach einem der Ansprüche 1 bis 10, die die folgende Gleichung erfüllt:

$$0{,}0178\ \text{Nb} + 0{,}0143\ \text{Mo} + 0{,}0243\ \text{Ta} + 0{,}0285\ \text{Sn} < 1{,}0,$$

in der Mo, Nb, Ta und Sn die Mengen an Molybdän, Niob, Tantal bzw. Zinn in Gew.% darstellen.

12. Legierungszusammensetzung auf Titanbasis nach einem der Ansprüche 1 bis 11, die die folgende Gleichung erfüllt:

$$0{,}0298\ \text{Nb} + 0{,}0272\ \text{Mo} + 0{,}0246\ \text{Ta} + 0{,}0376\ \text{Zr} + 0{,}0259\ \text{Sn} > 1{,}0,$$

bevorzugt

$$0{,}0298\ \text{Nb} + 0{,}0272\ \text{Mo} + 0{,}0246\ \text{Ta} + 0{,}0376\ \text{Zr} + 0{,}0259\ \text{Sn} > 1{,}1,$$

wobei Zr, Nb, Mo, Ta und Sn die Mengen an Zirconium, Niob, Molybdän, Tantal bzw. Zinn in Gew.-% darstellen.

13. Legierungszusammensetzung auf Titanbasis nach einem der Ansprüche 1 bis 12, (i) die entweder die folgende Gleichung erfüllt:

$$2,5 > 0,042 \, Nb + 0,06 \, Mo + 0,05 \, Ta + 0,03 \, Zr + 0,1 \, Sn,$$

in der Mo, Nb, Ta, Zr und Sn die Mengen an Molybdän, Niob, Tantal, Zirconium bzw. Zinn in Gew.-% darstellen, und/oder (ii) die die folgende Gleichung erfüllt:

$$0,042 \, Nb + 0,06 \, Mo + 0,05 \, Ta + 0,03 \, Zr + 0,1 \, Sn > 1,0,$$

in der Mo, Nb, Ta, Zr und Sn die Mengen an Molybdän, Niob, Tantal, Zirconium bzw. Zinn in Gew.-% darstellen.

**14.** Implantat oder Prothesenvorrichtung, das/die aus der Legierung auf Titanbasis nach einem der Ansprüche 1 bis 13 hergestellt ist.

**15.** Verfahren zur additiven Fertigung eines Artikels, das die Legierung auf Titanbasis nach einem der Ansprüche 1 bis 13 verwendet, bevorzugt wobei der Artikel ein Implantat oder eine Prothesenvorrichtung ist.

**Revendications**

**1.** Composition d'alliage à base de titane constituée en pourcentage en poids par : entre 15,0 et 35,0% de niobium, entre 0,0 et 7,5% de molybdène, entre 0,0 et 20,0% de tantale, entre 0 et 7,0% de zirconium, entre 0 et 6,0% d'étain, entre 0,0 et 2,0% d'hafnium, entre 0,0 et 0,5% d'aluminium, entre 0,0 et 0,5% de vanadium, entre 0,0 et 0,5% de fer, entre 0,0 et 0,5% de chrome, entre 0,0 et 0,5% de cobalt, entre 0,0 et 0,5% de nickel, entre 0,0 et 1,0% de silicium, entre 0,0 et 0,2% de bore, entre 0,0 et 0,5% de calcium, entre 0,0 et 0,5% de carbone, entre 0,0 et 0,5% de manganèse, entre 0,0 et 0,5% d'or, entre 0,0 et 0,5% d'argent, entre 0,0 et 0,5% d'oxygène, entre 0,0 et 0,5% d'hydrogène, entre 0,0 et 0,5% d'azote, entre 0,0 et 0,5% de palladium, entre 0,0 et 0,5% de lanthane, le complément étant du titane et des impuretés accidentelles, dans laquelle composition répond à l'équation suivante :

$$0,0175 \, Nb + 0,0183 \, Mo + 0,03 \, Ta + 0,0116 \, Zr + 0,1 \, Sn > 1,0$$

où Nb, Mo, Ta, Zr et Sn représentent les quantités de niobium, molybdène, tantale, zirconium et étain en % en poids respectivement.

**2.** Composition d'alliage à base de titane selon la revendication 1, constituée par 7,0% ou moins de molybdène, préférablement 6,0% ou moins de molybdène, plus préférablement 5,0 ou moins de molybdène.

**3.** Composition d'alliage à base de titane selon la revendication 1 ou 2, constituée par 1,5% ou plus de molybdène, préférablement 2,5% ou plus de molybdène.

**4.** Composition d'alliage à base de titane selon la revendication 1, 2, ou 3, qui satisfait l'équation suivante $0,0375 \, Nb + 0,033 \, Mo + 0,0167 \, Ta + 0,05 \, Zr + 0,267 - (0,13 Sn - 0,516)^2 > 1,0$, préférablement > 1,1 où Zr, Sn, Mo, Ta et Nb représentent respectivement les quantités de zirconium, d'étain, de molybdène, de tantale, et de niobium en % en poids.

**5.** Composition d'alliage à base de titane selon l'une quelconque des revendications 1 à 4, qui est soit (i) constituée par 5,5% ou moins d'étain, préférablement 5,0% ou moins d'étain, plus préférablement 4,75% ou moins d'étain et/ou (ii) constituée par 2,0% ou plus d'étain, préférablement 3,0% ou plus d'étain, plus préférablement 4,0% ou plus d'étain.

**6.** Composition d'alliage à base de titane selon l'une quelconque des revendications 1 à 5, qui est soit (i) constituée par 6,0% ou moins de zirconium, préférablement 5,5% ou moins de zirconium, plus préférablement 4,5% ou moins de zirconium, encore plus préférablement 4,0% ou moins zirconium, le plus préférablement 3,5% ou moins de zirconium et/ou (ii) constitué par 1,0% ou plus de zirconium, préférablement 1,5% ou plus de zirconium, plus préférablement 2,0% ou plus de zirconium.

**7.** Composition d'alliage à base de titane selon l'une quelconque des revendications 1 à 6, qui satisfait l'équation suivante

$$1,0 \leq \text{Al \% en poids} + 1/3\ \text{Sn \% en poids} + 1/6\ \text{Zr \% en poids} \leq 2,5$$

où Al, Sn et Zr représentent respectivement les quantités d'aluminium, d'étain et de zirconium en % en poids.

8. Composition d'alliage à base de titane selon l'une quelconque des revendications 1 à 7, qui est soit (i) constituée par 17,5% ou plus de niobium, préférablement 20,0% ou plus de niobium, plus préférablement 22,5% ou plus de niobium et/ou (ii) constituée par 32,5% ou moins de niobium, préférablement 30,0% ou moins de niobium, plus préférablement 27,5% ou moins de niobium.

9. Composition d'alliage à base de titane selon l'une quelconque des revendications 1 à 8, qui est soit (i) constituée par 17,5% ou moins de tantale, préférablement 15,0% ou moins de tantale, plus préférablement 12,5% ou moins de tantale et/ou (ii) constituée par 5,0% ou plus de tantale, préférablement 7,5% ou plus de tantale, plus préférablement 10,0% ou plus de tantale, le plus préférablement 12,5% ou plus de tantale.

10. Composition d'alliage à base de titane selon l'une quelconque des revendications 1 à 9, soit (i) qui satisfait l'équation suivante

250 ≥ 883 - 150 Fe % en poids - 96 Cr % en poids - 49 Mo % en poids - 37 V en poids % - 17 Nb % en poids - 12 Ta % en poids - 7 Zr % en poids - 3 Sn % en poids + 15 Al % en poids, préférablement 225 ≥ 883 - 150 Fe % en poids - 96 Cr % en poids - 49 Mo % en poids - 37 V % en poids - 17 Nb % en poids - 12 Ta % en poids - 7 Zr % en poids - 3 Sn % en poids + 15 Al % en poids, plus préférablement 200 ≥ 883 - 150 Fe % en poids - 96 Cr % en poids - 49 Mo % en poids - 37 V % en poids - 17 Nb % en poids - 12 Ta % en poids - 7 Zr % en poids - 3 Sn % en poids + 15 Al en poids %
dans laquelle Fe, Cr, Mo, V, Nb, Ta, Zr, Sn et Al représentent respectivement les quantités de fer, chrome, molybdène, vanadium, niobium, tantale, zirconium, étain et aluminium en % en poids et/ou qui satisfait l'équation suivante
75 ≤ 883 - 150 Fe % en poids - 96 Cr % en poids - 49 Mo % en poids - 37 V % en poids - 17 Nb % en poids -12 Ta % en poids - 7 Zr % en poids - 3 Sn % en poids + 15 Al % en poids ≤ 250 dans laquelle Fe, Cr, Mo, V, Nb, Ta, Zr, Sn et Al représentent respectivement les quantités de fer, chrome, molybdène, vanadium, niobium, tantale, zirconium, étain et aluminium en % en poids.

11. Composition d'alliage à base de titane selon l'une quelconque des revendications 1 à 10, qui satisfait l'équation suivante

$$0,0178\ \text{Nb} + 0,0143\ \text{Mo} + 0,0243\ \text{Ta} + 0,0285\ \text{Sn} < 1,0$$

dans laquelle Mo, Nb, Ta et Sn représentent les quantités de molybdène, niobium, tantale et étain en % en poids respectivement.

12. Composition d'alliage à base de titane selon l'une quelconque des revendications 1 à 11, qui satisfait l'équation suivante

$$0,0298\ \text{Nb} + 0,0272\ \text{Mo} + 0,0246\ \text{Ta} + 0,0376\ \text{Zr} + 0,0259\ \text{Sn} > 1,0,$$

préférablement 0,0298 Nb + 0,0272 Mo + 0,0246 Ta + 0,0376 Zr + 0,0259 Sn > 1,1 où Zr, Nb, Mo, Ta et Sn représentent les quantités de zirconium, niobium, molybdène, tantale et étain en % poids respectivement.

13. Composition d'alliage à base de titane selon l'une quelconque des revendications 1 à 12, soit (i) qui satisfait à l'équation suivante
2,5 > 0,042 Nb + 0,06 Mo + 0,05 Ta + 0,03 Zr + 0,1 Sn dans laquelle Mo, Nb, Ta, Zr et Sn représentent les quantités de molybdène, niobium, tantale, zirconium et étain en % en poids respectivement ; et/ou (ii) qui satisfait l'équation suivante

$$0,042 \, \text{Nb} + 0,06 \, \text{Mo} + 0,05 \, \text{Ta} + 0,03 \, \text{Zr} + 0,1 \, \text{Sn} > 1,0$$

dans laquelle Mo, Nb, Ta, Zr et Sn représentent les quantités de molybdène, niobium, tantale, zirconium et étain en % en poids respectivement.

14. Implant ou dispositif prothétique constitué par l'alliage à base de titane selon l'une quelconque des revendications 1 à 13.

15. Procédé de fabrication additive d'un article qui utilise l'alliage à base de titane selon l'une quelconque des revendications 1 à 13, préférablement dans lequel l'article est un implant ou un dispositif prothétique.

# Fig. 1

Bio-compatibility of various elements of common alloy elements. Obtained from Kuroda D, Niinomi M, Morinaga M, Kato Y, Yashiro T. Design and mechanical properties of new beta type titanium alloys for implant materials. Materials Science and Engineering: A. 1998.

# Fig. 2

Flow diagram of the alloy by design process.

```
┌──────────────────────────────┐        ┌──────────────────┐
│  Propose alloy space search  │ ─────▶ │     Table 2      │
└──────────────────────────────┘        └──────────────────┘
                │
                ▼
┌──────────────────────────────┐
│ Run thermodynamic calculations│
└──────────────────────────────┘
                │
                ▼
┌──────────────────────────────┐
│     Propose merit indeces    │
└──────────────────────────────┘
```

| Manufacturability | Cracking | Bio-compatibility | Elastic modulus | Strength |
|---|---|---|---|---|

```
┌──────────────────────────────┐        ┌──────────────────┐
│  Contour mapping the merit   │ ─────▶ │  Figures 3 to 9  │
│  indeces for the alloy space │        └──────────────────┘
└──────────────────────────────┘
                │
                ▼
┌──────────────────────────────┐        ┌──────────────────┐
│     Isolate effect of elements│ ────▶ │    Figure 10     │
│       on the merit indeces   │        └──────────────────┘
└──────────────────────────────┘
                │
                ▼
┌──────────────────────────────┐        ┌──────────────────┐
│ Allication of the merit-force-approach│ ─▶ │    Figure 11     │
│  to isolate optimal compositions│     └──────────────────┘
└──────────────────────────────┘
                │
                ▼
┌──────────────────────────────┐        ┌──────────────────┐
│     Optimal alloy range      │ ─────▶ │  Figures 12 to 26│
└──────────────────────────────┘        └──────────────────┘
```

Fig. 3   Cracking susceptibility factor contours for the Ti-Nb-Mo-Ta-Zr system with Sn=0.

Cracking susceptibility (-)

Fig. 3 (Cont.)

EP 3 759 258 B1

# Fig. 4

Solidification (freezing) range contours for the Ti-Nb-Mo-Ta-Zr system with Sn=0.

Solidification (freezing) range (°C)

# Fig. 5

Cost contours for the Ti-Nb-Mo-Ta-Zr system with Sn=0.

Raw material cost ($/ton)

# Fig. 6

Martensitic start temperature contours for the Ti-Nb-Mo-Ta-Zr system with Sn=0.
Martensitic start temperature (°C)

# Fig. 7

Bond order contours for the Ti-Nb-Mo-Ta-Zr system with Sn=0.

Bond order (-)

EP 3 759 258 B1

Fig. 8    Growth restriction factor contours for the Ti-Nb-Mo-Ta-Zr system with Sn=0.

Growth restriction factor (-)

Fig. 8 (Cont.)

EP 3 759 258 B1

# Fig. 9

Melting temperature contours for the Ti-Nb-Mo-Ta-Zr system with Sn=0.

Melting temperature (°C)

# Fig. 10

Qualitative effect of alloying elements on merit indeces.

| Factor | Nb | Ta | Zr | Mo | Sn | Al |
|---|---|---|---|---|---|---|
| CSC (lower is better) | ↓↓ | ↓ | ↓ | ↓ | ↓↑ | ↑ |
| GRF (higher is better) | ↑↑ | ↑ | ↑ | ↑↑ | ↑↑↑ | - |
| Bo (higher is better) | ↑ | ↑↑ | ↑ | ↑ | ↓ | ↓↓ |
| $M_S$ (optimal range 100-300°C) | ↓↓ | ↓ | ↓ | ↓↓↓ | ↓ | ↑↑ |
| Solidification range (lower is better - but not critical) | ↑↑ | ↑ | ↑ | ↑↑ | ↑ | - |
| Liquidus temperature (lower is better) | ↑ | ↑ | - | ↑ | - | - |
| Strength (higher is better) | ↓ | ↓ | ↑ | - | ↑ | ↑↑↑ |
| Bio-compatibility (higher is better) | ↑↑ | ↑↑↑ | ↑↑ | - | ↑ | ↓↓↓ |
| Cost (lower is better) | ↑↑ | ↑↑↑ | ↑↑↑ | ↑ | ↑ | ↓↓ |

EP 3 759 258 B1

# Fig. 11

Constrains used to find the optimal alloy space.

## Five-forces design

**Strength by heat-treatability**
Constrain: Mtemp > 75°C
Martensitic temperature
Merit Index

**Strength by grain refinement**
Constrain: GRF > 150
Growth Restriction Factor
Merit Index

**Bio-compatibility**
Constrain: Only bio-compatible elements & Bo > 2.85
Cell growth and elasticity merit index

Optimal alloy space

**Cracking susceptibility**
CSF < 0.33
Mtemp < 250°C
GRF > 150
Crack Susceptibility Factor
Growth Restriction Factor
Martensitic temperature

Scheil range < 250°C
Melting < 1900°C
Ti > 50 wt. %

Solidification range, melting temperature and Ti wt. %

**Powder production and additive manufacturing stability**

# Fig. 12

Contour map of the freezing range (°C) for a selected Ti-Nb-Mo-Ta-XSn-YZr system.

Solidification (freezing) range (°C)

# Fig. 13

Contour map of the cracking susceptibility factor (-) for a selected
Ti-Nb-Mo- Ta-XSn-YZr system.

Cracking susceptibility (-)

# Fig. 14

Contour map of the growth restriction factor (-) for a
selected Ti-Nb-Mo-Ta-XSn-YZr system.

Growth restriction factor (-)

Fig. 15    Contour map of the melting temperature temperature for a selected Ti-Nb-Mo-Ta-XSn-YZr system.

Fig. 15 (Cont.)

# Fig. 16

Contour map of the martensitic start temperature (°C) for a
selected Ti-Nb-Mo-Ta-XSn-YZr system.

Martensitic start temperature (°C)

EP 3 759 258 B1

# Fig. 17

Contour map of the raw material cost ($/ton) for a
selected Ti-Nb-Mo-Ta-XSn-YZr system.

Raw material cost ($/ton)

# Fig. 18

Contour map of the bond order (-) for a selected Ti-Nb-Mo-Ta-XSn-YZr system.

Bond order (-)

# Fig. 19

Contour map of the aluminium equivalent value for the Ti-Nb-Mo-Ta-XSn-YZr system.

Aluminium equivalent (wt. %)

# Fig. 20

Contour map of the optimal alloy space for the Ti-Nb-Mo-Ta-XSn-YZr system
as limited by the introduced thresholds.

Property thresholds

## Fig. 21 — Values of the introduced thresholds and comparison to actual values for example and baseline alloys.

### Property thresholds

**Thresholds for optimal alloy space**

| Num. | Index | |
|---|---|---|
| 1 | Al eq. | $1 < Sn/3 + Zr/6 < 2.5$ |
| 2 | GRF | $0.0175Nb + 0.0183Mo + 0.03Ta + 0.0116Zr + 0.1Sn > 1.0$ |
| 3a | CSF | $0.0375Nb + 0.033Mo + 0.0167Ta + 0.05Zr + 0.267 - (0.13Sn - 0.516)^2 > 1.0$ |
| 3b | CSF | $Zr/4 < 1.0$ |
| 4 | Melting | $0.0178Nb + 0.0143Mo + 0.0243Ta + 0.0285Sn < 1.0$ |
| 5 | Bo | $0.0298Nb + 0.0272Mo + 0.0246Ta + 0.0376Zr + 0.0259Sn > 1.0$ |
| 6-7 | Ms | $75 > 883-150Fe-49Mo - 17Nb - 12Ta - 7Zr - 3Sn < 250$ |
| 8 | Range | $2.5 > 0.042Nb + 0.06Mo + 0.05Ta + 0.03Zr +0.1Sn > 1.0$ |

**Actual design restrictions**

| | |
|---|---|
| GRF | >150 |
| CSF | <0.33 |
| Melting | <1900 |
| Bo | >2.85 |
| Ms | 75>Ms<250 |
| Range | 75>Range<250 |

☐ Within threshold  
▦ Excluded

| Baseline | | Nb | Mo | Ta | Zr | Sn | Fe | Hf | 1 Al eq | 2 GRF | Actual GRF | 3a CSF | 3b CSF | Actual CSF | 4 Melting | Actual Melting | 5 Bo | Actual Bo | 6-7 Ms | Actual Ms | 8 Range | Actual Range |
|---|---|---|---|---|---|---|---|---|---|---|---|---|---|---|---|---|---|---|---|---|---|---|
| 1 | | 13 | 0 | 0 | 13 | 0 | 0 | 0 | 2.60 | 0.38 | 20 | -0.29 | 3.25 | 1.11 | 0.23 | 1662 | 0.88 | 2.84 | 571 | 571 | 0.94 | 62 |
| 2 | * | 0 | 12 | 0 | 6 | 0 | 2 | 0 | 1.20 | 0.29 | 53 | 0.13 | 1.50 | 3.06 | 0.17 | 1680 | 0.55 | 2.82 | -47 | -47 | 0.90 | 580 |
| 3 | * | 0 | 15 | 0 | 5 | 0 | 0 | 0 | 1.00 | 0.33 | 30 | 0.29 | 1.25 | 0.90 | 0.21 | 1735 | 0.60 | 2.80 | 158 | 158 | 1.05 | 78 |
| 4 | * | 3 | 12 | 0 | 0 | 0 | 0 | 0 | 0.00 | 0.27 | 29 | 0.51 | 0.00 | 0.51 | 0.23 | 1726 | 0.42 | 2.81 | 244 | 244 | 0.85 | 49 |
| 5 | * | 0 | 12 | 5 | 0 | 0 | 0 | 0 | 0.00 | 0.37 | 39 | 0.51 | 0.00 | 0.48 | 0.29 | 1741 | 0.45 | 2.81 | 235 | 235 | 0.97 | 64 |
| 6 | * | 16 | 0 | 0 | 0 | 0 | 0 | 10 | 0.00 | 0.28 | 15 | 0.45 | 0.00 | 0.52 | 0.29 | 1699 | 0.48 | 2.83 | 611 | 611 | 0.67 | 24 |
| 7 | * | 35 | 0 | 5 | 7 | 0 | 0 | 0 | 1.40 | 0.84 | 122 | 0.71 | 1.75 | 0.48 | 0.75 | 1834 | 1.43 | 2.88 | 179 | 179 | 1.93 | 210 |
| 8 | | 29 | 0 | 13 | 4.6 | 0 | 0 | 0 | 0.92 | 0.95 | 124 | 0.80 | 1.15 | 0.45 | 0.83 | 1850 | 1.36 | 2.88 | 202 | 202 | 2.01 | 206 |
| 9 | | 28 | 0 | 0 | 13 | 0 | 0.5 | 0 | 2.60 | 0.64 | 94 | 0.13 | 3.25 | 2.32 | 0.50 | 1733 | 1.33 | 2.87 | 241 | 241 | 1.57 | 369 |
| 10 | * | 24 | 0 | 0 | 4 | 7.9 | 0 | 0 | 3.06 | 1.26 | 109 | 0.48 | 1.00 | 0.76 | 0.65 | 1740 | 0.66 | 2.82 | 423 | 423 | 1.92 | 111 |
| 11 | * | 0 | 0 | 30 | 0 | 0 | 0 | 0 | 0.00 | 0.90 | 63 | 0.50 | 0.00 | 0.47 | 0.73 | 1792 | 0.74 | 2.83 | 523 | 523 | 1.50 | 115 |
| 12 | * | 0 | 0 | 50 | 0 | 0 | 0 | 0 | 0.00 | 1.50 | 110 | 0.83 | 0.00 | 0.55 | 1.21 | 1912 | 1.23 | 2.86 | 283 | 283 | 2.50 | 226 |
| 13 | * | 18 | 2 | 10 | 0 | 0 | 0 | 0 | 0.00 | 0.65 | 73 | 0.74 | 0.00 | 0.36 | 0.59 | 1777 | 0.84 | 2.84 | 359 | 359 | 1.38 | 98 |
| 14 | | 29 | 4 | 13 | 0 | 0 | 0 | 0 | 0.00 | 0.97 | 143 | 1.17 | 0.00 | 0.31 | 0.89 | 1895 | 1.30 | 2.88 | 38 | 38 | 2.11 | 211 |
| 15 | | 16 | 7 | 13 | 0 | 0 | 0 | 0 | 0.00 | 0.80 | 111 | 0.91 | 0.00 | 0.34 | 0.70 | 1827 | 0.99 | 2.84 | 112 | 112 | 1.74 | 146 |
| 16 | | 16 | 4 | 13 | 0 | 0 | 0 | 0 | 0.00 | 0.74 | 90 | 0.81 | 0.00 | 0.36 | 0.66 | 1801 | 0.91 | 2.84 | 259 | 259 | 1.56 | 121 |

# Fig. 21 (Cont.)

| Examples | Nb | Mo | Ta | Zr | Sn | Fe | Hf | 1 Al eq | 2 GRF | Actual GRF | 3a CSF | 3b CSF | Actual CSF | 4 Melting | Actual Melting | 5 Bo | Actual Bo | 6-7 Ms | Actual Ms | 8 Range | Actual Range |
|---|---|---|---|---|---|---|---|---|---|---|---|---|---|---|---|---|---|---|---|---|---|
| 1 | 18.5 | 4 | 17 | 2.7 | 4.5 | 0 | 0 | 1.83 | 1.39 | 231 | 1.07 | 0.68 | 0.33 | 0.93 | 1884 | 1.07 | 2.85 | 136 | 136 | 2.40 | 234 |
| 2 | 18.5 | 1.5 | 19 | 2.7 | 4.5 | 0 | 0 | 1.83 | 1.40 | 188 | 1.01 | 0.68 | 0.33 | 0.94 | 1859 | 1.05 | 2.85 | 235 | 235 | 2.35 | 212 |
| 3 | 20 | 5.3 | 14 | 2.7 | 4.5 | 0 | 0 | 1.83 | 1.33 | 218 | 1.10 | 0.68 | 0.32 | 0.89 | 1875 | 1.06 | 2.85 | 91 | 91 | 2.36 | 230 |
| 4 | 22.5 | 4.8 | 13 | 2.5 | 4.5 | 0 | 0 | 1.79 | 1.34 | 219 | 1.14 | 0.63 | 0.30 | 0.90 | 1882 | 1.09 | 2.86 | 87 | 87 | 2.38 | 236 |
| 5 | 25 | 4.5 | 11 | 3 | 4.5 | 0 | 0 | 1.89 | 1.32 | 221 | 1.15 | 0.75 | 0.31 | 0.89 | 1884 | 1.12 | 2.86 | 77 | 77 | 2.39 | 245 |
| 6 | 27.5 | 3.5 | 8.5 | 2.5 | 4.5 | 0 | 0 | 1.79 | 1.28 | 210 | 1.17 | 0.63 | 0.29 | 0.88 | 1875 | 1.10 | 2.86 | 111 | 111 | 2.32 | 230 |
| 7 | 30 | 3 | 7.5 | 2.5 | 4.5 | 0 | 0 | 1.79 | 1.28 | 208 | 1.21 | 0.63 | 0.28 | 0.89 | 1882 | 1.14 | 2.86 | 105 | 105 | 2.34 | 238 |
| 8 | 32.5 | 2.5 | 6.5 | 2.5 | 4.5 | 0 | 0 | 1.79 | 1.29 | 204 | 1.24 | 0.63 | 0.27 | 0.90 | 1888 | 1.18 | 2.87 | 99 | 99 | 2.37 | 247 |
| 9 | 35 | 2 | 5.5 | 2 | 4.5 | 0 | 0 | 1.69 | 1.29 | 196 | 1.30 | 0.50 | 0.25 | 0.92 | 1894 | 1.19 | 2.87 | 97 | 96 | 2.38 | 248 |

**EP 3 759 258 B1**

# Fig. 22

Scatter plots of the growth restriction factor vs. the solidification range. Area highlighted in black indicates the optimal design space. Labelled data points correspond to literature alloys. Light grey highlights whole search space.

47

# Fig. 23

Scatter plots of the cracking susceptibility factor vs. the solidification range.
Area highlighted in black indicates the optimal design space. Labelled data points
correspond to literature alloys. Light grey highlights whole search space.

# Fig. 24

Scatter plots of the growth restriction factor vs. the cracking susceptibility factor. Area highlighted in black indicates the optimal design space. Labelled data points correspond to literature alloys. Light grey highlights whole search space.

# Fig. 25

Scatter plots of the growth restriction factor vs. other merit indeces. Area highlighted in black indicates the optimal design space. White data points correspond to literature alloys. Light grey highlights whole search space.

# Fig. 26

Scatter plots of the cracking susceptibility factor vs. other merit indeces.
Area highlighted in black indicates the optimal design space. White data points
correspond to literature alloys. Light grey highlights whole search space.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 105734312 A

### Non-patent literature cited in the description

- **E. L. PANG et al.** The effect of zirconium on the omega phase in Ti-24Nb [0-8] Zr alloys. *Acta Materialia,* vol. 153, 62-70
- **KURODA D ; NIINOMI M ; MORINAGA M ; KATO Y ; YASHIRO T.** Design and mechanical properties of new β type titanium alloys for implant materials. *Materials Science and Engineering: A,* 1998
- **NUNOME.** In vitro evaluation of biocompatibility of Ti-Mo-Sn-Zr superelastic alloy. *Journal of Biomaterials Applications,* 2015
- **OKAZAKI ; TETSUYA.** Corrosion resistance, mechanical properties: corrosion fatigue strength and cytocompatibility of new Ti alloys without Al and V. *Biomaterials,* 1998
- **BROWN SA ; LEMONS JE.** *Medical Applications of Titanium and Its Alloys: The Material and Biological Issues,* 1996
- **SURESH NEELAKANTANA.** Prediction of the martensite start temperature for β titanium alloys as a function of composition. *Scripta Materialia,* 2009, vol. 60, 611
- **T. E. QUESTED ; A. T. DINSDALE ; A. L. GREER.** Thermodynamic Modeling of Growth-Restriction Effects in Aluminum Alloys. *Acta Materialia,* 2005, vol. 53, 1323